# EUROPEAN PATENT SPECIFICATION

(11) **EP 3 370 708 B1**
(45) Date of publication and mention of the grant of the patent: **02.12.2026**
(21) Application number: 16863162.0
(22) Date of filing: 07.11.2016
(51) Int. Cl.: C12N 15/113, A61K 31/7125

(54) **MODULATING APOLIPOPROTEIN (a) EXPRESSION**
MODULATION DER APOLIPOPROTEIN (A)-EXPRESSION
MODULATION DE L'EXPRESSION DE L'APOLIPOPROTÉINE (A)

(30) Priority: 06.11.2015 US 201562252392 P
(43) Date of publication of application: 12.09.2018
(73) Proprietor: Ionis Pharmaceuticals, Inc., Carlsbad, CA 92010 (US)
(72) Inventor: VINEY, Nicholas, J., Carlsbad, CA 92010 (US); GEARY, Richard, S., Carlsbad, CA 92010 (US); WANG, Yanfeng, Carlsbad, CA 92010 (US); YU, Zhengrong, Carlsbad, CA 92010 (US); GUNAWAN, Rudy, Carlsbad, CA 92010 (US)
(74) Representative: Leitner, Laura
(86) International application number: PCT/US2016/060816
(87) International publication number: WO 2017/079739

(56) References cited:
- WO-A2-2015/168589
- US-A1- 2015 126 719
- US-A1- 2015 126 720
- US-A1- 2015 126 720
- SOTIRIOS TSIMIKAS ET AL: "Antisense therapy targeting apolipoprotein(a): a randomised, double-blind, placebo-controlled phase 1 study", LANCET, vol. 386, no. 10002, 1 October 2015 (2015-10-01), AMSTERDAM, NL, pages 1472 - 1483, XP055535698, ISSN: 0140-6736, DOI: 10.1016/S0140-6736(15)61252-1

## Description

### SEQUENCE LISTING

The present application is being filed along with a Sequence Listing in electronic format. The Sequence Listing is provided as a file BIOL0283WOSEQ_ST25.txt, created on November 3, 2016, which is 4 Kb in size.

### BACKGROUND OF THE INVENTION

The principle behind antisense technology is that an antisense compound hybridizes to a target nucleic acid and modulates the amount, activity, and/or function of the target nucleic acid. For example in certain instances, antisense compounds result in altered transcription or translation of a target. Such modulation of expression can be achieved by, for example, target mRNA degradation or occupancy-based inhibition. An example of modulation of RNA target function by degradation is RNase H-based degradation of the target RNA upon hybridization with a DNA-like antisense compound. Another example of modulation of gene expression by target degradation is RNA interference (RNAi). RNAi refers to antisense-mediated gene silencing through a mechanism that utilizes the RNA-induced siliencing complex (RISC). An additional example of modulation of RNA target function is by an occupancy-based mechanism such as is employed naturally by microRNA. MicroRNAs are small non-coding RNAs that regulate the expression of protein-coding RNAs. The binding of an antisense compound to a microRNA prevents that microRNA from binding to its messenger RNA targets, and thus interferes with the function of the microRNA. MicroRNA mimics can enhance native microRNA function. Certain antisense compounds alter splicing of pre-mRNA. Regardless of the specific mechanism, sequence-specificity makes antisense compounds attractive as tools for target validation and gene functionalization, as well as therapeutics to selectively modulate the expression of genes involved in the pathogenesis of diseases.

Antisense technology is an effective means for modulating the expression of one or more specific gene products and can therefore prove to be uniquely useful in a number of therapeutic, diagnostic, and research applications. Chemically modified nucleosides may be incorporated into antisense compounds to enhance one or more properties, such as nuclease resistance, pharmacokinetics or affinity for a target nucleic acid. In 1998, the antisense compound, Vitravene^{®} (fomivirsen; developed by Isis Pharmaceuticals Inc., Carlsbad, CA) was the first antisense drug to achieve marketing clearance from the U.S. Food and Drug Administration (FDA), and is currently a treatment of cytomegalovirus (CMV)-induced retinitis in AIDS patients.

New chemical modifications have improved the potency and efficacy of antisense compounds, uncovering the potential for oral delivery as well as enhancing subcutaneous administration, decreasing potential for side effects, and leading to improvements in patient convenience. Chemical modifications increasing potency of antisense compounds allow administration of lower doses, which reduces the potential for toxicity, as well as decreasing overall cost of therapy. Modifications increasing the resistance to degradation result in slower clearance from the body, allowing for less frequent dosing. Different types of chemical modifications can be combined in one compound to further optimize the compound's efficacy.

Lipoproteins are globular, micelle-like particles that consist of a non-polar core of acylglycerols and cholesteryl esters surrounded by an amphiphilic coating of protein, phospholipid and cholesterol. Lipoproteins have been classified into five broad categories on the basis of their functional and physical properties: chylomicrons, very low density lipoproteins (VLDL), intermediate density lipoproteins (IDL), low density lipoproteins (LDL), and high density lipoproteins (HDL). Chylomicrons transport dietary lipids from intestine to tissues. VLDLs, IDLs and LDLs all transport triacylglycerols and cholesterol from the liver to tissues. HDLs transport endogenous cholesterol from tissues to the liver

Lipoprotein particles undergo continuous metabolic processing and have variable properties and compositions. Lipoprotein densities increase without increasing particle diameter because the density of their outer coatings is less than that of the inner core. The protein components of lipoproteins are known as apolipoproteins. At least nine apolipoproteins are distributed in significant amounts among the various human lipoproteins.

The lipoprotein(a) [Lp(a)] particle was identified nearly 50 years ago and is comprised of a highly unique LDL particle in which one apolipoprotein B (apoB) protein is linked via a disulfide bond to a single apolipoprotein(a) [apo(a)] protein. The apo(a) protein shares a high degree of homology with plasminogen particularly within the kringle IV type 2 repetitive domain. Levels of circulating Lp(a) are inversely proportional to the number of kringle IV type 2 variable repeats present in the molecule and, as both alleles are co-expressed within individuals, can display heterozygous plasma isoform profiles (Kraft et al., Eur J Hum Genet, 1996; 4(2): 74-87). It is thought that this kringle repeat domain in apo(a) may be responsible for its pro-thrombotic and anti-fibrinolytic properties, potentially enhancing atherosclerotic progression.

Apo(a) is transcriptionally regulated by IL-6 and in studies in rheumatoid arthritis patients treated with an IL-6 inhibitor (tocilizumab), plasma levels were reduced by 30% after 3 month treatment (Schultz et al., PLoS One 2010; 5:e14328).

Apo(a) has been shown to preferentially bind oxidized phospholipids and potentiate vascular inflammation (Bergmark et al., J Lipid Res 2008; 49:2230-2239; Tsimikas et al., Circulation. 2009; 119(13):1711-1719).

Further, studies suggest that the Lp(a) particle may also stimulate endothelial permeability, induce plasminogen activator inhibitor type-1 expression and activate macrophage interleukin-8 secretion (Koschinsky and Marcovina, Curr Opin Lipidol 2004; 15:167-174). Importantly, recent genetic association studies revealed that Lp(a) was an independent risk factor for myocardial infarction, stroke, peripheral vascular disease and abdominal aortic aneurysm (Rifai et al., Clin Chem 2004; 50:1364-71; Erqou et al., JAMA 2009;302:412-23; Kamstrup et al., Circulation 2008;117:176-84). Further, in the recent Precocious Coronary Artery Disease (PROCARDIS) study, Clarke *et al.* (Clarke et al., NEJM (2009)361; 2518-2528) described robust and independent associations between coronary heart disease and plasma Lp(a) concentrations. Additionally, Solfrizzi et al., suggested that increased serum Lp(a) may be linked to an increased risk for Alzheimer's Disease (AD) (Solfrizzi et al., J Neurol Neurosurg Psychiatry 2002, 72:732-736. Currently, in the clinic setting, examples of indirect apo(a) inhibitors for treating cardiovascular disease include aspirin, Niaspan, Mipomersen, Anacetrapib, Epirotirome and Lomitapide which reduce plasma Lp(a) levels by 18%, 39%, 32%, 36%, 43% and 17%, respectively. Additionally, Lp(a) apheresis has been used in the clinic to reduce apo(a) containing Lp(a) particles.

To date, therapeutic strategies to treat cardiovascular disease by directly targeting apo(a) levels have been limited. Ribozyme oligonucleotides (U.S. Patent 5,877,022) and antisense oligonucleotides (WO 2005/000201; WO 2003/014397; WO2013/177468; US20040242516; U.S. Patent Nos. 8,138,328, 8,673,632 and 7,259,150; Merki et al., J Am Coll Cardiol 2011; 57:1611-1621) have been developed but none have been approved for commercial use.

WO 2014/179625 discloses antisense compounds targeting apo(a), including ISIS 681257.

Tsimikas et al. (Lancet. 2015 Oct 10; 386:1472-83) discloses the results of a randomized, double-blind, placebo-controlled Phase 1 study using an antisense compound targeting human apo(a): ISIS 494372 (also known as ISIS-APO(a)Rx).

One chemical modification used to improve the activity of RNAse H dependent (gapmer) antisense compounds, including Apo(a) targeting compounds, *in vivo* is conjugation to a conjugate group, such as a GalNAc cluster. Conjugation to a conjugate group has been shown to improve potency in vivo in non-human subjects, for example including the use of RNAse H dependent (gapmer) antisense compounds conjugated to GalNAc clusters as disclosed in WO 2014/179620. Prior to the present invention, no RNAse H dependent (gapmer) antisense compounds conjugated to GalNAc clusters had been tested in humans to achieve target reduction.

There remains an unmet medical need for novel agents which can potently and selectively reduce apo(a) levels in humans, including in patients at enhanced risk for cardiovascular events due to chronically elevated plasma Lp(a) levels.

### SUMMARY OF THE INVENTION

The present invention provides the oligomeric compound ISIS 681257 for use in treating or preventing a disease or condition in a human, especially a human at enhanced risk for cardiovascular events due to chronically elevated plasma Lp(a) levels, wherein from 75 mg to 85 mg of the oligomeric compound are administered to the human during a dosing period of one month.

The present invention also provides the oligomeric compound ISIS 681257 for use in treating or preventing a disease or condition in a human, especially a human at enhanced risk for cardiovascular events due to chronically elevated plasma Lp(a) levels, wherein 80 mg of the oligomeric compound is administered to a human once every month.

ISIS 681257 has the following structure, which includes salts thereof:

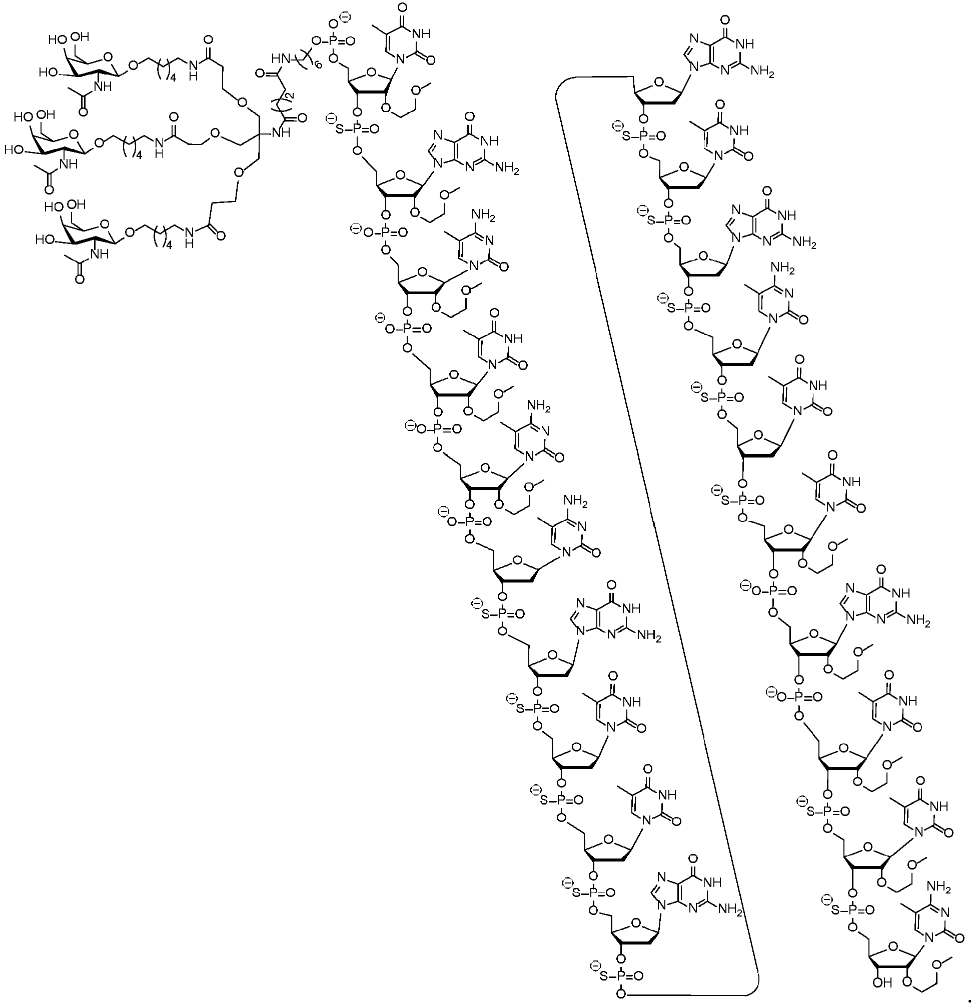

Pictured below is an example of a salt of ISIS 681257:

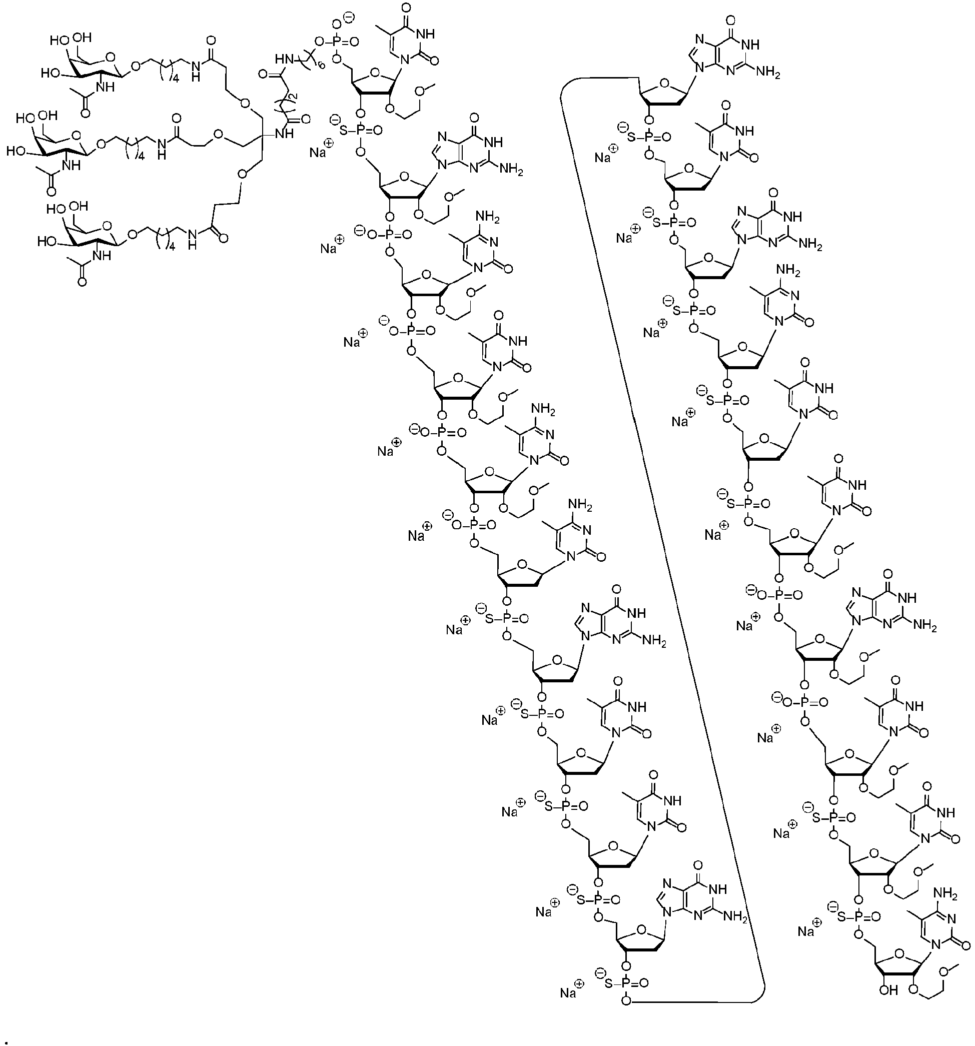

ISIS 681257 comprises a modified oligonucleotide having the nucleobase sequence TGCTCCGTTGGTGCTTGTTC (SEQ ID NO.: 1), a 5-10-5 gapmer motif, and a GalNAc conjugate. As illustrated in the present disclosure, ISIS 681257 represents a major advance in treating humans. When administered to humans, ISIS 681257 is particularly efficacious at lowering Apo(a) mRNA and plasma Lp(a), in terms of both its potency and its duration of action. As illustrated by the data provided herein, ISIS 681257 has ≥ 30-fold increase in humans compared to a modified oligonucleotide having the same nucleobase sequence and the same 5-10-5 gapmer motif, but lacking a GalNAc conjugate. ISIS 681257 provides excellent reduction of Apo(a) mRNA and plasma Lp(a) and enables efficacious dosing of once a week, once a month, once every two months, or once every three months.

The clinical results presented herein for ISIS 681257 are surprising, because earlier experiments involving both the unconjugated compound (ISIS 494372 also having the nucleobase sequence TGCTCCGTTGGTGCTTGTTC (SEQ ID NO.: 1) and a 5-10-5 gapmer motif, and the GalNAc conjugated compound (ISIS 681257) had suggested that the GalNAc conjugated compound would have significantly lower potency and/or a shorter duration of action in humans than was observed following the first dosing of humans reported herein (*e.g.* see Examples 89, 100 and 108 of WO 2014/179625 and Tsimikas et al., Lancet, 2015 Oct 10; 386:1472-83). Earlier experiments involving both ISIS 494372 and ISIS 681257 had indicated that the GalNAc conjugated compound benefits from higher *in vivo* potency in mice, but these earlier experiments did not reveal or predict the unexpected ≥ 30-fold improvement in humans. In earlier experiments ISIS 681257 was found to have around 13-fold improved potency (on baseline plasma Lp(a) levels) relative to ISIS 494372 in human transgenic Apo(a) mice (ISIS 681257 ED50 = 0.8 mg/kg/wk; ISIS 494372 ED50 = 11 mg/kg/wk). The fold improvement in potency between ISIS 494372 and ISIS 681257 observed in the human Phase 1 clinical trials was surprisingly over 2 times higher than the fold improvement observed for the same pair of compounds in mice, and this was unexpected. In light of these surprising results, when treating humans, the GalNAc conjugated compound (ISIS 681257 and its salts) can be administered at lower doses and/or less frequently than expected based on the earlier *in vivo* testing of the GalNAc conjugated compound. *See, e.g.,* Viney, et al. Lancet, 2016, Sep 2016; 388: 2239-53. This can provide one or more very significant improvements in treating humans, e.g. reduced cost of treatment, improved patient compliance, reduced volume of administered medicinal product and/or potentially reduced risk of potential adverse events via lower dose administration regimens.

The present disclosure provides the following non-limiting embodiments:
In one embodiment of the invention, the treatment comprises administering a unit dose comprising not more than 75mg of the oligomeric compound.

In one embodiment of the invention, the treatment comprises administering a unit dose comprising not more than 50mg of the oligomeric compound.

In one embodiment of the invention, the treatment comprises administering a unit dose comprising not more than 25mg of the oligomeric compound.

In one embodiment of the invention, the treatment comprises administering a unit dose comprising not more than 15mg of the oligomeric compound.

In one embodiment of the invention, the treatment comprises administering a unit dose comprising not less than 1mg of the oligomeric compound.

In one embodiment of the invention, the treatment comprises administering a unit dose comprising not less than 2.5mg of the oligomeric compound

In one embodiment of the invention, the treatment comprises administering a unit dose comprising not less than 5mg of the oligomeric compound.

In one embodiment of the invention, the treatment comprises administering a unit dose of from 75mg to 85 mg, and optionally a unit dose of 80mg.

In one embodiment of the invention, n the treatment comprises administering a unit dose of from 55mg to 65 mg, and optionally a unit dose of 60mg.

In one embodiment of the invention, the treatment comprises administering a unit dose of from 35mg to 45mg, and optionally a unit dose of 40mg.

In one embodiment of the invention, the treatment comprises administering a unit dose of from 25mg to 35mg, and optionally a unit dose of 30mg.

In one embodiment of the invention, the treatment comprises administering a unit dose of from 15mg to 25 mg, and optionally a unit dose of 20mg.

In one embodiment of the invention, the treatment comprises administering a unit dose of from 5mg to 15mg, and optionally a unit dose of 10mg.

In one embodiment of the invention, the treatment comprises administering not more than 1 unit dose to the human during the dosing period.

In one embodiment of the invention, the use comprises administering not more than 2 unit doses to the human during the dosing period.

In one embodiment of the invention, the treatment comprises administering not more than 3 unit doses to the human during the dosing period.

In one embodiment of the invention, the treatment comprises administering not more than 4 unit doses to the human during the dosing period.

In one embodiment of the invention, the treatment comprises administering not more than 5 unit doses to the human during the dosing period.

In one embodiment of the invention, the treatment comprises administering not more than 6 unit doses to the human during the dosing period.

In one embodiment of the invention, the oligomeric compound is for use in treating or preventing a disease or condition in a human, wherein (i) the treatment comprises administering from 75mg to 85 mg, optionally 80mg, of the oligomeric compound to the human during the dosing period; (ii) the dosing period is one month; and (iii) the treatment comprises administering not more than one unit dose to the human during the dosing period.

In one embodiment of the invention, the human is at enhanced risk for cardiovascular events due to elevated plasma Lp(a) levels.

In one embodiment of the invention, the disease or condition is selected from calcific aortic valve stenosis with elevated Lp(a), elevated cardiovascular risk with elevated Lp(a), and recurrent cardiovascular events with elevated Lp(a).

In one embodiment of the invention, the oligomeric compound is administered to the human by injection.

In one embodiment of the invention, the oligomeric compound is administered to the human by subcutaneous injection.

In one embodiment of the invention, the oligomeric compound is formulated in a sterile liquid and optionally wherein each unit dose of the oligomeric compound is not more than 1 mL of the sterile liquid.

In one embodiment of the invention, each unit dose of the oligomeric compound is not more than 0.8 mL of the sterile liquid.

In one embodiment of the invention, each unit dose of the oligomeric compound is not more than 0.5 mL of the sterile liquid.

In one embodiment of the invention, each unit dose of the oligomeric compound is not more than 0.4 mL of the sterile liquid.

In one embodiment of the invention, each unit dose of the oligomeric compound is not more than 0.25 mL of the sterile liquid.

In one embodiment of the invention, each unit dose of the oligomeric compound is not more than 0.2 mL of the sterile liquid.

In one embodiment of the invention, the sterile liquid is water.

In one embodiment of the invention, the sterile liquid is water with a sodium phosphate buffer.

In one embodiment of the invention, the sterile liquid is water with a sodium phosphate buffer and sodium chloride.

In one embodiment of the invention,the treatment reduces the fasting plasma Lp(a) concentration in the human by at least 50%, when the fasting plasma Lp(a) concentration in the human is measured at the start and end of the dosing period.

In one embodiment of the invention, the treatment reduces the fasting plasma Lp(a) concentration in the human by at least 75%, when the fasting plasma Lp(a) concentration in the human is measured at the start and end of the dosing period

In one embodiment of the invention, the treatment reduces the fasting plasma Lp(a) concentration in the human by at least 80%, when the fasting plasma Lp(a) concentration in the human is measured at the start and end of the dosing period.

In one embodiment of the invention, the treatment reduces the fasting plasma Lp(a) concentration in the human by at least 85%, when the fasting plasma Lp(a) concentration in the human is measured at the start and end of the dosing period.

In certain embodiments, the present disclosure provides an oligomeric compound, wherein the oligomeric compound is ISIS 681257, for use in treating or preventing a disease or condition associated with elevated Lp(a) in a human as claimed herein, wherein the treatment comprises administering one or more doses of the oligomeric compound to the human in (a) a loading or induction phase, and (b) a maintenance phase.

The present disclosure provides ISIS 681257 for use as claimed herein for administering to a patient in need thereof. In certain embodiments, a patient in need thereof is a human with elevated Apo(a) levels, for example, a human having apo(a) levels ≥ 30 mg/dL, ≥ 35 mg/dL, ≥ 40 mg/dL, ≥ 50 mg/dL, ≥ 60 mg/dL, ≥ 70 mg/dL, ≥ 80 mg/dL, ≥ 90 mg/dL, ≥ 100 mg/dL, ≥ 110 mg/dL, ≥ 120 mg/dL, ≥ 130 mg/dL, ≥ 140 mg/dL, ≥ 150 mg/dL, ≥ 160 mg/dL, ≥ 170 mg/dL, ≥ 175 mg/dL, ≥ 180 mg/dL, ≥ 190 mg/dL, ≥ 200 mg/dL. Lp(a) may also be expressed in nanomoles per liter. For example, a human subject having ≥ 75 nanomoles/liter (nmol/L) or ≥ 30 mg/dL, would be considered at risk of one or more symptoms of a cardiovascular disease or disorder.

### BRIEF DESCRIPTION OF THE FIGURES

The following Figures 1A-C, 2A, 3, 4, 5A-C, and 6A, 6B, and 6D refer to Examples that are not according to the invention and are present for illustration purposes only.
Figures 1A-C illustrate the predicted Lp(a) levels as a result of different weekly dosing regimens. Doses of 20 mg (Fig 1A), 30 mg (Fig 1B) or 40 mg (Fig 1C) shows a steady state reduction of Lp (a) of ≥ 80%.
Figures 2A-B illustrate the predicted Lp(a) levels as a result of different monthly dosing regimens. Doses of 60 mg (Fig 2A) and 80 mg (Fig 2B) Lp(a) show a steady state reduction of Lp (a) of about 80%.
Figure 3 illustrates the predicted Lp (a) levels as a result of a 2-month dosing regimen (e.g. one dose every two months). An 80 mg dose every 2-months shows a steady state reduction of Lp (a) of about 80%.
Figure 4 illustrates the predicted Lp (a) levels as a result of a quarterly dosing regimen. An 80 mg dose every quarter shows a steady state reduction of Lp (a) of 80% and maximum reduction of Lp (a) of > 90%.
Figures 5A-D illustrate the predicted Lp(a) levels as a result of different monthly dosing regimens. Figures are shown modeling the effect on Lp(a) by monthly administration of ISIS 681257 at doses of 20 mg (Fig 5A), 40 mg (Fig. 5B), 60 mg (Fig. 5C), and 80 mg (Fig.5D ). The dark middle line represents the predicted dose, while the uppermost and lowermost lines represent the 90% Confidence Interval.
Figures 6A-D illustrate the predicted Lp(a) levels as a result of different weekly dosing regimens. Figures 6A-D show modeling of the effect on Lp(a) by weekly administration of ISIS 681257 at doses of 5 mg (Fig 6A), 10 mg (Fig. 6B), 20 mg (Fig. 6C), and 30 mg (Fig. 6D). The dark middle line represents the predicted dose, while the uppermost and lowermost lines represent the 90% Confidence Interval.

### DETAILED DESCRIPTION

It is to be understood that both the foregoing general description and the following detailed description are exemplary and explanatory only and are not restrictive of the disclosure. Herein, the use of the singular includes the plural unless specifically stated otherwise. As used herein, the use of "or" means "and/or" unless stated otherwise. Furthermore, the use of the term "including" as well as other forms, such as "includes" and "included", is not limiting. Also, terms such as "element" or "component" encompass both elements and components comprising one unit and elements and components that comprise more than one subunit, unless specifically stated otherwise.

The section headings used herein are for organizational purposes only and are not to be construed as limiting the subject matter described.

### A. Definitions

Unless specific definitions are provided, the nomenclature used in connection with, and the procedures and techniques of, analytical chemistry, synthetic organic chemistry, and medicinal and pharmaceutical chemistry described herein are those well known and commonly used in the art. Standard techniques may be used for chemical synthesis, and chemical analysis. Certain such techniques and procedures may be found for example in "Carbohydrate Modifications in Antisense Research" Edited by Sangvi and Cook, American Chemical Society , Washington D.C., 1994; "Remington's Pharmaceutical Sciences," Mack Publishing Co., Easton, Pa., 21st edition, 2005; and "Antisense Drug Technology, Principles, Strategies, and Applications" Edited by Stanley T. Crooke, CRC Press, Boca Raton, Florida; and Sambrook et al., "Molecular Cloning, A laboratory Manual," 2nd Edition, Cold Spring Harbor Laboratory Press, 1989.

Unless otherwise indicated, the following terms have the following meanings:
As used herein, "dosing period" means the period of time between when a human subject receives the first dose and when the human subject receives a final dose. It is envisaged that dosing of the patient may continue after the end of the dosing period, such that a first dosing period is followed by one or more further dosing periods during which the same of a different dosing regimen is used. For example, a human subject may receive 6 doses in a first dosing period where the first and last dose are given 4 weeks apart. Subsequently, the human subject may then start a second dosing period where the human subject receives doses at regular intervals (e.g. one unit dose per week, one unit dose per month, or one unit dose per quarter).

As used herein, the term "unit dose" refers to the specific amount of the oligomeric compound administered to the human at a particular time point (*e.g.* the specific amount of the oligomeric compound administered to the human in a single subcutaneous injection). Each unit dose forms part of a multi-dose regimen, as described herein.

As used herein, the term "unit dosage form" denotes the physical form in which each unit dose is presented for administration.

As used here, the term "sterile liquid" means and liquid suitable for administration to a human subject. In certain embodiments, sterile liquids comprise liquids that are substantially free from viable microorganisms or bacteria. In certain embodiments, sterile liquids comprise USP grade water or USP grade saline.

As used herein, "nucleoside" means a compound comprising a nucleobase moiety and a sugar moiety. Nucleosides include, but are not limited to, naturally occurring nucleosides (as found in DNA and RNA) and modified nucleosides. Nucleosides may be linked to a phosphate moiety.

As used herein, "chemical modification" means a chemical difference in a compound when compared to a naturally occurring counterpart. Chemical modifications of oligonucleotides include nucleoside modifications (including sugar moiety modifications and nucleobase modifications) and internucleoside linkage modifications. In reference to an oligonucleotide, chemical modification does not include differences only in nucleobase sequence.

As used herein, "furanosyl" means a structure comprising a 5-membered ring comprising four carbon atoms and one oxygen atom.

As used herein, "naturally occurring sugar moiety" means a ribofuranosyl as found in naturally occurring RNA or a deoxyribofuranosyl as found in naturally occurring DNA.

As used herein, "sugar moiety" means a naturally occurring sugar moiety or a modified sugar moiety of a nucleoside.

As used herein, "modified sugar moiety" means a substituted sugar moiety or a sugar surrogate.

As used herein, "substituted sugar moiety" means a furanosyl that is not a naturally occurring sugar moiety. Substituted sugar moieties include, but are not limited to furanosyls comprising substituents at the 2'-position, the 3'-position, the 5'-position and/or the 4'-position. Certain substituted sugar moieties are bicyclic sugar moieties.

As used herein, "2'-substituted sugar moiety" means a furanosyl comprising a substituent at the 2'-position other than H or OH. Unless otherwise indicated, a 2'-substituted sugar moiety is not a bicyclic sugar moiety (i.e., the 2'-substituent of a 2'-substituted sugar moiety does not form a bridge to another atom of the furanosyl ring.

As used herein, "MOE" means -OCH₂CH₂OCH₃.

As used herein, "nucleic acid" refers to molecules composed of monomeric nucleotides. A nucleic acid includes ribonucleic acids (RNA), deoxyribonucleic acids (DNA), single-stranded nucleic acids (ssDNA), double-stranded nucleic acids (dsDNA), small interfering ribonucleic acids (siRNA), and microRNAs (miRNA). A nucleic acid may also comprise any combination of these elements in a single molecule.

As used herein, "nucleotide" means a nucleoside further comprising a phosphate linking group. As used herein, "linked nucleosides" may or may not be linked by phosphate linkages and thus includes, but is not limited to "linked nucleotides." As used herein, "linked nucleosides" are nucleosides that are connected in a continuous sequence (i.e. no additional nucleosides are present between those that are linked).

As used herein, "nucleobase" means a group of atoms that can be linked to a sugar moiety to create a nucleoside that is capable of incorporation into an oligonucleotide, and wherein the group of atoms is capable of bonding with a complementary naturally occurring nucleobase of another oligonucleotide or nucleic acid. Nucleobases may be naturally occurring or may be modified. As used herein, "nucleobase sequence" means the order of contiguous nucleobases independent of any sugar, linkage, or nucleobase modification.

As used herein the terms, "unmodified nucleobase" or "naturally occurring nucleobase" means the naturally occurring heterocyclic nucleobases of RNA or DNA: the purine bases adenine (A) and guanine (G), and the pyrimidine bases thymine (T), cytosine (C) (including 5-methyl C), and uracil (U).

As used herein, "modified nucleobase" means any nucleobase that is not a naturally occurring nucleobase.

As used herein, "modified nucleoside" means a nucleoside comprising at least one chemical modification compared to naturally occurring RNA or DNA nucleosides. Modified nucleosides comprise a modified sugar moiety and/or a modified nucleobase.

As used herein, "2'-substituted nucleoside" means a nucleoside comprising a substituent at the 2'-position other than H or OH. Unless otherwise indicated, a 2'-substituted nucleoside is not a bicyclic nucleoside.

As used herein, "deoxynucleoside" means a nucleoside comprising 2'-H furanosyl sugar moiety, as found in naturally occurring deoxyribonucleosides (DNA). In certain embodiments, a 2' -deoxynucleoside may comprise a modified nucleobase or may comprise an RNA nucleobase (e.g., uracil).

As used herein, "oligonucleotide" means a compound comprising a plurality of linked nucleosides. In certain embodiments, an oligonucleotide comprises one or more unmodified ribonucleosides (RNA) and/or unmodified deoxyribonucleosides (DNA) and/or one or more modified nucleosides.

As used herein "oligonucleoside" means an oligonucleotide in which none of the internucleoside linkages contains a phosphorus atom. As used herein, oligonucleotides include oligonucleosides.

As used herein, "modified oligonucleotide" means an oligonucleotide comprising at least one modified nucleoside and/or at least one modified internucleoside linkage.

As used herein, "linkage" or "linking group" means a group of atoms that link together two or more other groups of atoms.

As used herein "internucleoside linkage" means a covalent linkage between adjacent nucleosides in an oligonucleotide.

As used herein "naturally occurring internucleoside linkage" means a 3' to 5' phosphodiester linkage.

As used herein, "modified internucleoside linkage" means any internucleoside linkage other than a naturally occurring internucleoside linkage.

As used herein, "terminal internucleoside linkage" means the linkage between the last two nucleosides of an oligonucleotide or defined region thereof.

As used herein, "phosphorus linking group" means a linking group comprising a phosphorus atom. Phosphorus linking groups include without limitation groups having the formula: 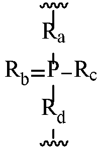 wherein:
Rₐ and R_{d} are each, independently, O, S, CH₂, NH, or NJ₁ wherein J₁ is C₁-C₆ alkyl or substituted C₁-C₆ alkyl;
R_{b} is O or S;
R_{c} is OH, SH, C₁-C₆ alkyl, substituted C₁-C₆ alkyl, C₁-C₆ alkoxy, substituted C₁-C₆ alkoxy, amino or substituted amino; and
J₁ is R_{b} is O or S.

Phosphorus linking groups include without limitation, phosphodiester, phosphorothioate, phosphorodithioate, phosphonate, phosphoramidate, phosphorothioamidate, thionoalkylphosphonate, phosphotriesters, thionoalkylphosphotriester and boranophosphate.

As used herein, "internucleoside phosphorus linking group" means a phosphorus linking group that directly links two nucleosides.

As used herein, "non-internucleoside phosphorus linking group" means a phosphorus linking group that does not directly link two nucleosides. In certain embodiments, a non-internucleoside phosphorus linking group links a nucleoside to a group other than a nucleoside. In certain embodiments, a non-internucleoside phosphorus linking group links two groups, neither of which is a nucleoside.

As used herein, "neutral linking group" means a linking group that is not charged. Neutral linking groups include without limitation phosphotriesters, methylphosphonates, MMI (-CH₂-N(CH₃)-O-), amide-3 (-CH₂-C(=O)-N(H)-), amide-4 (-CH₂-N(H)-C(=O)-), formacetal (-O-CH₂-O-), and thioformacetal (-S-CH₂-O-). Further neutral linking groups include nonionic linkages comprising siloxane (dialkylsiloxane), carboxylate ester, carboxamide, sulfide, sulfonate ester and amides (See for example: Carbohydrate Modifications in Antisense Research; Y.S. Sanghvi and P.D. Cook Eds. ACS Symposium Series 580; Chapters 3 and 4, (pp. 40-65)). Further neutral linking groups include nonionic linkages comprising mixed N, O, S and CH₂ component parts.

As used herein, "internucleoside neutral linking group" means a neutral linking group that directly links two nucleosides.

As used herein, "oligomeric compound" means a polymeric structure comprising two or more substructures. In certain embodiments, an oligomeric compound comprises an oligonucleotide. In certain embodiments, an oligomeric compound comprises one or more conjugate groups and/or terminal groups. In certain embodiments, an oligomeric compound consists of an oligonucleotide. Oligomeric compounds also include naturally occurring nucleic acids. In certain embodiments, an oligomeric compound comprises a backbone of one or more linked monomeric subunits where each linked monomeric subunit is directly or indirectly attached to a heterocyclic base moiety. In certain embodiments, oligomeric compounds may also include monomeric subunits that are not linked to a heterocyclic base moiety, thereby providing abasic sites. In certain embodiments, the linkages joining the monomeric subunits, the sugar moieties or surrogates and the heterocyclic base moieties can be independently modified. In certain embodiments, the linkage-sugar unit, which may or may not include a heterocyclic base, may be substituted with a mimetic such as the monomers in peptide nucleic acids.

As used herein, "terminal group" means one or more atom attached to either, or both, the 3' end or the 5' end of an oligonucleotide. In certain embodiments a terminal group is a conjugate group. In certain embodiments, a terminal group comprises one or more terminal group nucleosides.

As used herein, "conjugate" or "conjugate group" means an atom or group of atoms bound to an oligonucleotide or oligomeric compound. In general, conjugate groups modify one or more properties of the compound to which they are attached, including, but not limited to pharmacodynamic, pharmacokinetic, binding, absorption, cellular distribution, cellular uptake, charge and/or clearance properties.

As used herein, "conjugate linker" or "linker" in the context of a conjugate group means a portion of a conjugate group comprising any atom or group of atoms and which covalently link (1) an oligonucleotide to another portion of the conjugate group or (2) two or more portions of the conjugate group.

Conjugate groups are shown herein as radicals, providing a bond for forming covalent attachment to an oligomeric compound such as an antisense oligonucleotide. In certain embodiments, the point of attachment on the oligomeric compound is the 3'-oxygen atom of the 3'-hydroxyl group of the 3' terminal nucleoside of the oligomeric compound. In certain embodiments the point of attachment on the oligomeric compound is the 5'-oxygen atom of the 5'-hydroxyl group of the 5' terminal nucleoside of the oligomeric compound. In certain embodiments, the bond for forming attachment to the oligomeric compound is a cleavable bond. In certain such embodiments, such cleavable bond constitutes all or part of a cleavable moiety.

In certain embodiments, conjugate groups comprise a cleavable moiety (e.g., a cleavable bond or cleavable nucleoside) and a carbohydrate cluster portion, such as a GalNAc cluster portion. Such carbohydrate cluster portion comprises: a targeting moiety and, optionally, a conjugate linker. In certain embodiments, the carbohydrate cluster portion is identified by the number and identity of the ligand. For example, in certain embodiments, the carbohydrate cluster portion comprises 3 GalNAc groups and is designated "GalNAc₃". Specific carbohydrate cluster portions (having specific tether, branching and conjugate linker groups) are described herein and designated by Roman numeral followed by subscript "a". Accordingly "GalNac3-1ₐ" refers to a specific carbohydrate cluster portion of a conjugate group having 3 GalNac groups and specifically identified tether, branching and linking groups. Such carbohydrate cluster fragment is attached to an oligomeric compound via a cleavable moiety, such as a cleavable bond or cleavable nucleoside.

As used herein, "cleavable moiety" means a bond or group that is capable of being split under physiological conditions. In certain embodiments, a cleavable moiety is cleaved inside a cell or sub-cellular compartments, such as a lysosome. In certain embodiments, a cleavable moiety is cleaved by endogenous enzymes, such as nucleases. In certain embodiments, a cleavable moiety comprises a group of atoms having one, two, three, four, or more than four cleavable bonds.

As used herein, "cleavable bond" means any chemical bond capable of being split. In certain embodiments, a cleavable bond is selected from among: an amide, a polyamide, an ester, an ether, one or both esters of a phosphodiester, a phosphate ester, a carbamate, a di-sulfide, or a peptide.

As used herein, "carbohydrate cluster" means a compound having one or more carbohydrate residues attached to a scaffold or linker group. (*see,* e.g., Maier et al., "Synthesis of Antisense Oligonucleotides Conjugated to a Multivalent Carbohydrate Cluster for Cellular Targeting," Bioconjugate Chemistry, 2003, (14): 18-29, or Rensen et al., "Design and Synthesis of Novel N-Acetylgalactosamine-Terminated Glycolipids for Targeting of Lipoproteins to the Hepatic Asiaglycoprotein Receptor," J. Med. Chem. 2004, (47): 5798-5808, for examples of carbohydrate conjugate clusters).

As used herein, "single-stranded" means an oligomeric compound that is not hybridized to its complement and which lacks sufficient self-complementarity to form a stable self-duplex.

As used herein, "double stranded" means a pair of oligomeric compounds that are hybridized to one another or a single self-complementary oligomeric compound that forms a hairpin structure. In certain embodiments, a double-stranded oligomeric compound comprises a first and a second oligomeric compound.

As used herein, "antisense compound" means a compound comprising or consisting of an oligonucleotide at least a portion of which is complementary to a target nucleic acid to which it is capable of hybridizing, resulting in at least one antisense activity.

As used herein, "antisense activity" means any detectable and/or measurable change attributable to the hybridization of an antisense compound to its target nucleic acid. In certain embodiments, antisense activity includes modulation of the amount or activity of a target nucleic acid transcript (e.g. mRNA). In certain embodiments, antisense activity includes modulation of the splicing of pre-mRNA.

As used herein, "RNase H based antisense compound" means an antisense compound wherein at least some of the antisense activity of the antisense compound is attributable to hybridization of the antisense compound to a target nucleic acid and subsequent cleavage of the target nucleic acid by RNase H.

As used herein, "detecting" or "measuring" means that a test or assay for detecting or measuring is performed. Such detection and/or measuring may result in a value of zero. Thus, if a test for detection or measuring results in a finding of no activity (activity of zero), the step of detecting or measuring the activity has nevertheless been performed.

As used herein, "detectable and/or measureable activity" means a statistically significant activity that is not zero.

As used herein, "essentially unchanged" means little or no change in a particular parameter, particularly relative to another parameter which changes much more. In certain embodiments, a parameter is essentially unchanged when it changes less than 5%. In certain embodiments, a parameter is essentially unchanged if it changes less than two-fold while another parameter changes at least ten-fold. For example, in certain embodiments, an antisense activity is a change in the amount of a target nucleic acid. In certain such embodiments, the amount of a non-target nucleic acid is essentially unchanged if it changes much less than the target nucleic acid does, but the change need not be zero.

As used herein, "expression" means the process by which a gene ultimately results in a protein. Expression includes, but is not limited to, transcription, post-transcriptional modification (e.g., splicing, polyadenlyation, addition of 5'-cap), and translation.

As used herein, "target nucleic acid" means a nucleic acid molecule to which an antisense compound is intended to hybridize to result in a desired antisense activity. Antisense oligonucleotides have sufficient complementarity to their target nucleic acids to allow hybridization under physiological conditions.

As used herein, "nucleobase complementarity" or "complementarity" when in reference to nucleobases means a nucleobase that is capable of base pairing with another nucleobase. For example, in DNA, adenine (A) is complementary to thymine (T). For example, in RNA, adenine (A) is complementary to uracil (U). In certain embodiments, complementary nucleobase means a nucleobase of an antisense compound that is capable of base pairing with a nucleobase of its target nucleic acid. For example, if a nucleobase at a certain position of an antisense compound is capable of hydrogen bonding with a nucleobase at a certain position of a target nucleic acid, then the position of hydrogen bonding between the oligonucleotide and the target nucleic acid is considered to be complementary at that nucleobase pair. Nucleobases comprising certain modifications may maintain the ability to pair with a counterpart nucleobase and thus, are still capable of nucleobase complementarity.

As used herein, "non-complementary" in reference to nucleobases means a pair of nucleobases that do not form hydrogen bonds with one another.

As used herein, "complementary" in reference to oligomeric compounds (e.g., linked nucleosides, oligonucleotides, or nucleic acids) means the capacity of such oligomeric compounds or regions thereof to hybridize to another oligomeric compound or region thereof through nucleobase complementarity. Complementary oligomeric compounds need not have nucleobase complementarity at each nucleoside. Rather, some mismatches are tolerated. In certain embodiments, complementary oligomeric compounds or regions are complementary at 70% of the nucleobases (70% complementary). In certain embodiments, complementary oligomeric compounds or regions are 80% complementary. In certain embodiments, complementary oligomeric compounds or regions are 90% complementary. In certain embodiments, complementary oligomeric compounds or regions are 95% complementary. In certain embodiments, complementary oligomeric compounds or regions are 100% complementary.

As used herein, "mismatch" means a nucleobase of a first oligomeric compound that is not capable of pairing with a nucleobase at a corresponding position of a second oligomeric compound, when the first and second oligomeric compound are aligned. Either or both of the first and second oligomeric compounds may be oligonucleotides.

As used herein, "hybridization" means the pairing of complementary oligomeric compounds (e.g., an antisense compound and its target nucleic acid). While not limited to a particular mechanism, the most common mechanism of pairing involves hydrogen bonding, which may be Watson-Crick, Hoogsteen or reversed Hoogsteen hydrogen bonding, between complementary nucleobases.

As used herein, "specifically hybridizes" means the ability of an oligomeric compound to hybridize to one nucleic acid site with greater affinity than it hybridizes to another nucleic acid site.

As used herein, "fully complementary" in reference to an oligonucleotide or portion thereof means that each nucleobase of the oligonucleotide or portion thereof is capable of pairing with a nucleobase of a complementary nucleic acid or contiguous portion thereof. Thus, a fully complementary region comprises no mismatches or unhybridized nucleobases in either strand.

As used herein, "percent complementarity" means the percentage of nucleobases of an oligomeric compound that are complementary to an equal-length portion of a target nucleic acid. Percent complementarity is calculated by dividing the number of nucleobases of the oligomeric compound that are complementary to nucleobases at corresponding positions in the target nucleic acid by the total length of the oligomeric compound.

As used herein, "percent identity" means the number of nucleobases in a first nucleic acid that are the same type (independent of chemical modification) as nucleobases at corresponding positions in a second nucleic acid, divided by the total number of nucleobases in the first nucleic acid.

As used herein, "modulation" means a change of amount or quality of a molecule, function, or activity when compared to the amount or quality of a molecule, function, or activity prior to modulation. For example, modulation includes the change, either an increase (stimulation or induction) or a decrease (inhibition or reduction) in gene expression. As a further example, modulation of expression can include a change in splice site selection of pre-mRNA processing, resulting in a change in the absolute or relative amount of a particular splice-variant compared to the amount in the absence of modulation.

As used herein, "chemical motif" means a pattern of chemical modifications in an oligonucleotide or a region thereof. Motifs may be defined by modifications at certain nucleosides and/or at certain linking groups of an oligonucleotide.

As used herein, "nucleoside motif" means a pattern of nucleoside modifications in an oligonucleotide or a region thereof. The linkages of such an oligonucleotide may be modified or unmodified. Unless otherwise indicated, motifs herein describing only nucleosides are intended to be nucleoside motifs. Thus, in such instances, the linkages are not limited.

As used herein, "sugar motif" means a pattern of sugar modifications in an oligonucleotide or a region thereof.

As used herein, "linkage motif" means a pattern of linkage modifications in an oligonucleotide or region thereof. The nucleosides of such an oligonucleotide may be modified or unmodified. Unless otherwise indicated, motifs herein describing only linkages are intended to be linkage motifs. Thus, in such instances, the nucleosides are not limited.

As used herein, "nucleobase modification motif" means a pattern of modifications to nucleobases along an oligonucleotide. Unless otherwise indicated, a nucleobase modification motif is independent of the nucleobase sequence.

As used herein, "sequence motif" means a pattern of nucleobases arranged along an oligonucleotide or portion thereof. Unless otherwise indicated, a sequence motif is independent of chemical modifications and thus may have any combination of chemical modifications, including no chemical modifications.

As used herein, "type of modification" in reference to a nucleoside or a nucleoside of a "type" means the chemical modification of a nucleoside and includes modified and unmodified nucleosides. Accordingly, unless otherwise indicated, a "nucleoside having a modification of a first type" may be an unmodified nucleoside.

As used herein, "differently modified" mean chemical modifications or chemical substituents that are different from one another, including absence of modifications. Thus, for example, a MOE nucleoside and an unmodified DNA nucleoside are "differently modified," even though the DNA nucleoside is unmodified. Likewise, DNA and RNA are "differently modified," even though both are naturally-occurring unmodified nucleosides. Nucleosides that are the same but for comprising different nucleobases are not differently modified. For example, a nucleoside comprising a 2'-OMe modified sugar and an unmodified adenine nucleobase and a nucleoside comprising a 2'-OMe modified sugar and an unmodified thymine nucleobase are not differently modified.

As used herein, "the same type of modifications" refers to modifications that are the same as one another, including absence of modifications. Thus, for example, two unmodified DNA nucleosides have "the same type of modification," even though the DNA nucleoside is unmodified. Such nucleosides having the same type modification may comprise different nucleobases.

As used herein, "separate regions" means portions of an oligonucleotide wherein the chemical modifications or the motif of chemical modifications of any neighboring portions include at least one difference to allow the separate regions to be distinguished from one another.

As used herein, "pharmaceutically acceptable carrier or diluent" means any substance suitable for use in administering to an animal. In certain embodiments, a pharmaceutically acceptable carrier or diluent is sterile saline. In certain embodiments, such sterile saline is pharmaceutical grade saline.

As used herein the term "metabolic disorder" means a disease or condition principally characterized by dysregulation of metabolism - the complex set of chemical reactions associated with breakdown of food to produce energy.

As used herein, the term "cardiovascular disorder" means a disease or condition principally characterized by impaired function of the heart or blood vessels.

As used herein, "prodrug" means an inactive or less active form of a compound which, when administered to a subject, is metabolized to form the active, or more active, compound (e.g., drug).

As used herein, unless otherwise indicated or modified, the term "double-stranded" refers to two separate oligomeric compounds that are hybridized to one another. Such double stranded compounds may have one or more or non-hybridizing nucleosides at one or both ends of one or both strands (overhangs) and/or one or more internal non-hybridizing nucleosides (mismatches) provided there is sufficient complementarity to maintain hybridization under physiologically relevant conditions.

As used herein, "5' target site" refers to the nucleotide of a target nucleic acid which is complementary to the 5'-most nucleotide of a particular antisense compound.

As used herein, "About" means within ±10% of a value. For example, if it is stated, "a marker may be increased by about 50%", it is implied that the marker may be increased between 45%-55%.

As used herein, "administered concomitantly" refers to the co-administration of two agents in any manner in which the pharmacological effects of both are manifest in the patient at the same time. Concomitant administration does not require that both agents be administered in a single pharmaceutical composition, in the same dosage form, or by the same route of administration. The effects of both agents need not manifest themselves at the same time. The effects need only be overlapping for a period of time and need not be coextensive.

As used herein, "administering" or "administration" means providing a pharmaceutical agent to an individual, and includes, but is not limited to, administering by a medical professional and self-administering. Administration of a pharmaceutical agent to an individual can be continuous, chronic, short or intermittent. Administration can parenteral or non-parenteral.

As used herein, "agent" means an active substance that can provide a therapeutic benefit when administered to an animal. "First agent" means a therapeutic compound of the invention. For example, a first agent can be an antisense oligonucleotide targeting apo(a). "Second agent" means a second therapeutic compound of the invention (e.g. a second antisense oligonucleotide targeting apo(a)) and/or a non-apo(a) therapeutic compound.

As used herein, "amelioration" or "ameliorate" or "ameliorating" refers to a lessening of at least one indicator, sign, or symptom of an associated disease, disorder, or condition. The severity of indicators can be determined by subjective or objective measures, which are known to those skilled in the art.

As used herein, "apo(a)" means any nucleic acid or protein sequence encoding apo(a). For example, in certain embodiments, apo(a) includes a DNA sequence encoding apo(a), a RNA sequence transcribed from DNA encoding apo(a) (including genomic DNA comprising introns and exons), a mRNA sequence encoding apo(a), or a peptide sequence encoding apo(a).

As used herein, "apo(a) nucleic acid" means any nucleic acid encoding apo(a). For example, in certain embodiments, an apo(a) nucleic acid includes a DNA sequence encoding apo(a), a RNA sequence transcribed from DNA encoding apo(a) (including genomic DNA comprising introns and exons), and a mRNA sequence encoding apo(a).

As used herein, "apo(a) mRNA" means a mRNA encoding an apo(a) protein.

As used herein, "apo(a) protein" means any protein sequence encoding Apo(a).

As used herein, "apo(a) specific inhibitor" refers to any agent capable of specifically inhibiting the expression of an apo(a) nucleic acid and/or apo(a) protein. For example, apo(a) specific inhibitors include nucleic acids (including antisense compounds), peptides, antibodies, small molecules, and other agents capable of inhibiting the expression of apo(a) nucleic acid and/or apo(a) protein. In certain embodiments, by specifically modulating apo(a) nucleic acid expression and/or apo(a) protein expression, apo(a) specific inhibitors can affect other components of the lipid transport system including downstream components. Similarly, in certain embodiments, apo(a) specific inhibitors can affect other molecular processes in an animal.

As used herein, "atherosclerosis" means a hardening of the arteries affecting large and medium-sized arteries and is characterized by the presence of fatty deposits. The fatty deposits are called "atheromas" or "plaques," which consist mainly of cholesterol and other fats, calcium and scar tissue, and damage the lining of arteries.

As used herein, "coronary heart disease (CHD)" means a narrowing of the small blood vessels that supply blood and oxygen to the heart, which is often a result of atherosclerosis.

As used herein, "diabetes mellitus" or "diabetes" is a syndrome characterized by disordered metabolism and abnormally high blood sugar (hyperglycemia) resulting from insufficient levels of insulin or reduced insulin sensitivity. The characteristic symptoms are excessive urine production (polyuria) due to high blood glucose levels, excessive thirst and increased fluid intake (polydipsia) attempting to compensate for increased urination, blurred vision due to high blood glucose effects on the eye's optics, unexplained weight loss, and lethargy.

As used herein, "diabetic dyslipidemia" or "type 2 diabetes with dyslipidemia" means a condition characterized by Type 2 diabetes, reduced HDL-C, elevated triglycerides (TG), and elevated small, dense LDL particles.

As used herein, "diluent" means an ingredient in a composition that lacks pharmacological activity, but is pharmaceutically necessary or desirable. For example, the diluent in an injected composition can be a liquid, e.g. saline solution.

As used herein, "dyslipidemia" refers to a disorder of lipid and/or lipoprotein metabolism, including lipid and/or lipoprotein overproduction or deficiency. Dyslipidemias can be manifested by elevation of lipids such as chylomicron, cholesterol and triglycerides as well as lipoproteins such as low-density lipoprotein (LDL) cholesterol.

As used herein, "dose" means a specified quantity of a pharmaceutical agent provided in a single administration, or in a specified time period. In certain embodiments, a dose can be administered in one, two, or more boluses, tablets, or injections. For example, in certain embodiments where subcutaneous administration is desired, the desired dose requires a volume not easily accommodated by a single injection, therefore, two or more injections can be used to achieve the desired dose. In certain embodiments, the pharmaceutical agent is administered by infusion over an extended period of time or continuously. Doses can be stated as the amount of pharmaceutical agent per hour, day, week, or month. Doses can also be stated as mg/kg or g/kg.

As used herein, "effective amount" or "therapeutically effective amount" means the amount of active pharmaceutical agent sufficient to effectuate a desired physiological outcome in an individual in need of the agent. The effective amount can vary among individuals depending on the health and physical condition of the individual to be treated, the taxonomic group of the individuals to be treated, the formulation of the composition, assessment of the individual's medical condition, and other relevant factors.

As used herein, "fully complementary" or "100% complementary" means each nucleobase of a nucleobase sequence of a first nucleic acid has a complementary nucleobase in a second nucleobase sequence of a second nucleic acid. In certain embodiments, a first nucleic acid is an antisense compound and a second nucleic acid is a target nucleic acid.

As used herein, "glucose" is a monosaccharide used by cells as a source of energy and inflammatory intermediate. "Plasma glucose" refers to glucose present in the plasma.

As used herein, "high density lipoprotein-C" or "HDL-C" means cholesterol associated with high density lipoprotein particles. Concentration of HDL-C in serum (or plasma) is typically quantified in mg/dL or nmol/L. "Serum HDL-C" and "plasma HDL-C" mean HDL-C in serum and plasma, respectively.

As used herein, "HMG-CoA reductase inhibitor" means an agent that acts through the inhibition of the enzyme HMG-CoA reductase, such as atorvastatin, rosuvastatin, fluvastatin, lovastatin, pravastatin, and simvastatin.

As used herein, "hypercholesterolemia" means a condition characterized by elevated cholesterol or circulating (plasma) cholesterol, LDL-cholesterol and VLDL-cholesterol, as per the guidelines of the Expert Panel Report of the National Cholesterol Educational Program (NCEP) of Detection, Evaluation of Treatment of high cholesterol in adults (see, Arch. Int. Med. (1988) 148, 36-39).

As used herein, "hyperlipidemia" or "hyperlipemia" is a condition characterized by elevated serum lipids or circulating (plasma) lipids. This condition manifests an abnormally high concentration of fats. The lipid fractions in the circulating blood are cholesterol, low density lipoproteins, very low density lipoproteins, chylomicrons and triglycerides. The Fredrickson classification of hyperlipidemias is based on the pattern of TG and cholesterol-rich lipoprotein particles, as measured by electrophoresis or ultracentrifugation and is commonly used to characterize primary causes of hyperlipidemias such as hypertriglyceridemia (Fredrickson and Lee, Circulation, 1965, 31:321-327; Fredrickson et al., New Eng J Med, 1967, 276 (1): 34-42).

As used herein, "hypertriglyceridemia" means a condition characterized by elevated triglyceride levels. Its etiology includes primary (i.e. genetic causes) and secondary (other underlying causes such as diabetes, metabolic syndrome/insulin resistance, obesity, physical inactivity, cigarette smoking, excess alcohol and a diet very high in carbohydrates) factors or, most often, a combination of both (Yuan et al. CMAJ, 2007, 176:1113-1120).

As used herein, "identifying" or "selecting an animal with metabolic or cardiovascular disease" means identifying or selecting a subject prone to or having been diagnosed with a metabolic disease, a cardiovascular disease, or a metabolic syndrome; or, identifying or selecting a subject having any symptom of a metabolic disease, cardiovascular disease, or metabolic syndrome including, but not limited to, hypercholesterolemia, hyperglycemia, hyperlipidemia, hypertriglyceridemia, hypertension increased insulin resistance, decreased insulin sensitivity, above normal body weight, and/or above normal body fat content or any combination thereof. Such identification can be accomplished by any method, including but not limited to, standard clinical tests or assessments, such as measuring serum or circulating (plasma) cholesterol, measuring serum or circulating (plasma) blood-glucose, measuring serum or circulating (plasma) triglycerides, measuring blood-pressure, measuring body fat content, measuring body weight, and the like.

As used herein, "improved cardiovascular outcome" means a reduction in the occurrence of adverse cardiovascular events, or the risk thereof. Examples of adverse cardiovascular events include, without limitation, death, reinfarction, stroke, cardiogenic shock, pulmonary edema, cardiac arrest, and atrial dysrhythmia.

As used herein, "increasing HDL" or "raising HDL" means increasing the level of HDL in an animal after administration of at least one compound of the invention, compared to the HDL level in an animal not administered any compound.

As used herein, "individual" or "subject" means a human selected for treatment or therapy.

As used herein, "individual in need thereof" refers to a human or non-human animal selected for treatment or therapy that is in need of such treatment or therapy.

As used herein, "induce", "inhibit", "potentiate", "elevate", "increase", "decrease", "reduce" or the like denote quantitative differences between two states. For example, "an amount effective to inhibit the activity or expression of apo(a)" means that the level of activity or expression of apo(a) in a treated sample will differ from the level of apo(a) activity or expression in an untreated sample. Such terms are applied to, for example, levels of expression, and levels of activity.

As used herein, "inflammatory condition" refers to a disease, disease state, syndrome, or other condition resulting in inflammation. For example, rheumatoid arthritis and liver fibrosis are inflammatory conditions. Other examples of inflammatory conditions include sepsis, myocardial ischemia/reperfusion injury, adult respiratory distress syndrome, nephritis, graft rejection, inflammatory bowel disease, multiple sclerosis, arteriosclerosis, atherosclerosis and vasculitis.

As used herein, "inhibiting the expression or activity" refers to a reduction or blockade of the expression or activity of a RNA or protein and does not necessarily indicate a total elimination of expression or activity.

As used herein, "insulin resistance" is defined as the condition in which normal amounts of insulin are inadequate to produce a normal insulin response from fat, muscle and liver cells. Insulin resistance in fat cells results in hydrolysis of stored triglycerides, which elevates free fatty acids in the blood plasma. Insulin resistance in muscle reduces glucose uptake whereas insulin resistance in liver reduces glucose storage, with both effects serving to elevate blood glucose. High plasma levels of insulin and glucose due to insulin resistance often leads to metabolic syndrome and type 2 diabetes.

As used herein, "insulin sensitivity" is a measure of how effectively an individual processes glucose. An individual having high insulin sensitivity effectively processes glucose whereas an individual with low insulin sensitivity does not effectively process glucose.

As used herein, "lipid-lowering" means a reduction in one or more lipids (e.g., LDL, VLDL) in a subject. "Lipid-raising" means an increase in a lipid (e.g., HDL) in a subject. Lipid-lowering or lipid-raising can occur with one or more doses over time.

As used herein, "lipid-lowering therapy" or "lipid lowering agent" means a therapeutic regimen provided to a subject to reduce one or more lipids in a subject. In certain embodiments, a lipid-lowering therapy is provided to reduce one or more of apo(a), CETP, apoB, total cholesterol, LDL-C, VLDL-C, IDL-C, non-HDL-C, triglycerides, small dense LDL particles, and Lp(a) in a subject. Examples of lipid-lowering therapy include, but are not limited to, apoB inhibitors, statins, fibrates and MTP inhibitors.

As used herein, "lipoprotein", such as VLDL, LDL and HDL, refers to a group of proteins found in the serum, plasma and lymph and are important for lipid transport. The chemical composition of each lipoprotein differs, for example, in that the HDL has a higher proportion of protein versus lipid, whereas the VLDL has a lower proportion of protein versus lipid.

As used herein, "Lp(a)" comprises apo(a) and a LDL like particle containing apoB. The apo(a) is linked to the apoB by a disulfide bond.

As used herein, "low density lipoprotein-cholesterol (LDL-C)" means cholesterol carried in low density lipoprotein particles. Concentration of LDL-C in serum (or plasma) is typically quantified in mg/dL or nmol/L. "Serum LDL-C" and "plasma LDL-C" mean LDL-C in the serum and plasma, respectively.

As used herein, "major risk factors" refers to factors that contribute to a high risk for a particular disease or condition. In certain embodiments, major risk factors for coronary heart disease include, without limitation, cigarette smoking, hypertension, high LDL, low HDL-C, family history of coronary heart disease, age, and other factors disclosed herein.

As used herein, "metabolic disorder" or "metabolic disease" refers to a condition characterized by an alteration or disturbance in metabolic function. "Metabolic" and "metabolism" are terms well known in the art and generally include the whole range of biochemical processes that occur within a living organism. Metabolic disorders include, but are not limited to, hyperglycemia, prediabetes, diabetes (type 1 and type 2), obesity, insulin resistance, metabolic syndrome and dyslipidemia due to type 2 diabetes.

As used herein, "metabolic syndrome" means a condition characterized by a clustering of lipid and non-lipid cardiovascular risk factors of metabolic origin. In certain embodiments, metabolic syndrome is identified by the presence of any 3 of the following factors: waist circumference of greater than 102 cm in men or greater than 88 cm in women; serum triglyceride of at least 150 mg/dL; HDL-C less than 40 mg/dL in men or less than 50 mg/dL in women; blood pressure of at least 130/85 mmHg; and fasting glucose of at least 110 mg/dL. These determinants can be readily measured in clinical practice (JAMA, 2001, 285: 2486-2497).

"Parenteral administration" means administration through injection or infusion. Parenteral administration includes subcutaneous administration, intravenous administration, intramuscular administration, intraarterial administration, intraperitoneal administration, or intracranial administration, e.g. intrathecal or intracerebroventricular administration. Administration can be continuous, chronic, short or intermittent.

As used herein, "pharmaceutical agent" means a substance that provides a therapeutic benefit when administered to an individual. For example, in certain embodiments, an antisense oligonucleotide targeted to apo(a) is a pharmaceutical agent.

As used herein, "pharmaceutical composition" or "composition" means a mixture of substances suitable for administering to an individual. For example, a pharmaceutical composition can comprise one or more active agents and a pharmaceutical carrier e.g., a sterile aqueous solution.

As used herein, "pharmaceutically acceptable derivative" encompasses derivatives of the compounds described herein such as solvates, hydrates, esters, prodrugs, polymorphs, isomers, isotopically labelled variants, pharmaceutically acceptable salts and other derivatives known in the art.

As used herein, "pharmaceutically acceptable salts" means physiologically and pharmaceutically acceptable salts of antisense compounds, i.e., salts that retain the desired biological activity of the parent compound and do not impart undesired toxicological effects thereto. The term "pharmaceutically acceptable salt" or "salt" includes a salt prepared from pharmaceutically acceptable non-toxic acids or bases, including inorganic or organic acids and bases. "Pharmaceutically acceptable salts" of the compounds described herein may be prepared by methods well-known in the art. For a review of pharmaceutically acceptable salts, see Stahl and Wermuth, Handbook of Pharmaceutical Salts: Properties, Selection and Use (Wiley-VCH, Weinheim, Germany, 2002). Sodium salts of antisense oligonucleotides are useful and are well accepted for therapeutic administration to humans. Accordingly, in one embodiment the compounds described herein are in the form of a sodium salt.

As used herein, "portion" means a defined number of contiguous (i.e. linked) nucleobases of a nucleic acid. In certain embodiments, a portion is a defined number of contiguous nucleobases of a target nucleic acid. In certain embodiments, a portion is a defined number of contiguous nucleobases of an antisense compound.

As used herein, "prevent"or "preventing" refers to delaying or forestalling the onset or development of a disease, disorder, or condition for a period of time from minutes to indefinitely. Prevent also means reducing risk of developing a disease, disorder, or condition.

As used herein, "raise" means to increase in amount. For example, to raise plasma HDL levels means to increase the amount of HDL in the plasma.

As used herein, "reduce" means to bring down to a smaller extent, size, amount, or number. For example, to reduce plasma triglyceride levels means to bring down the amount of triglyceride in the plasma.

As used herein, "region" or "target region" is defined as a portion of the target nucleic acid having at least one identifiable structure, function, or characteristic. For example, a target region may encompass a 3' UTR, a 5' UTR, an exon, an intron, an exon/intron junction, a coding region, a translation initiation region, translation termination region, or other defined nucleic acid region. The structurally defined regions for apo(a) can be obtained by accession number from sequence databases such as. In certain embodiments, a target region may encompass the sequence from a 5' target site of one target segment within the target region to a 3' target site of another target segment within the target region.

As used herein, "second agent" or "second therapeutic agent" means an agent that can be used in combination with a "first agent". A second therapeutic agent can include, but is not limited to, antisense oligonucleotides targeting apo(a) or apoB. A second agent can also include anti- apo(a) antibodies, apo(a) peptide inhibitors, cholesterol lowering agents, lipid lowering agents, glucose lowering agents and antiinflammatory agents.

As used herein, "segments" are defined as smaller, sub-portions of regions within a nucleic acid. For example, a "target segment" means the sequence of nucleotides of a target nucleic acid to which one or more antisense compounds is targeted. "5' target site" refers to the 5'-most nucleotide of a target segment. "3' target site" refers to the 3'-most nucleotide of a target segment. Alternatively, a "start site" can refer to the 5'-most nucleotide of a target segment and a "stop site" refers to the 3'-most nucleotide of a target segment. A target segment can also begin at the "start site" of one sequence and end at the "stop site" of another sequence.

As used herein, "statin" means an agent that inhibits the activity of HMG-CoA reductase.

As used herein, "subcutaneous administration" means administration just below the skin.

As used herein, "subject" means a human selected for treatment or therapy.

As used herein, "symptom of cardiovascular disease or disorder" means a phenomenon that arises from and accompanies the cardiovascular disease or disorder and serves as an indication of it. For example, angina; chest pain; shortness of breath; palpitations; weakness; dizziness; nausea; sweating; tachycardia; bradycardia; arrhythmia; atrial fibrillation; swelling in the lower extremities; cyanosis; fatigue; fainting; numbness of the face; numbness of the limbs; claudication or cramping of muscles; bloating of the abdomen; or fever are symptoms of cardiovascular disease or disorder.

As used herein, "targeting" or "targeted" means the process of design and selection of an antisense compound that will specifically hybridize to a target nucleic acid and induce a desired effect.

As used herein, "therapeutically effective amount" means an amount of a pharmaceutical agent that provides a therapeutic benefit to an individual.

As used herein, "therapeutic lifestyle change" means dietary and lifestyle changes intended to lower fat/adipose tissue mass and/or cholesterol. Such change can reduce the risk of developing heart disease, and may includes recommendations for dietary intake of total daily calories, total fat, saturated fat, polyunsaturated fat, monounsaturated fat, carbohydrate, protein, cholesterol, insoluble fiber, as well as recommendations for physical activity.

As used herein, "treat" or "treating" refers to administering a compound described herein to effect an alteration or improvement of a disease, disorder, or condition.

As used herein, "prevent" or "preventing" refers inhibit or delay one or more symptoms of a disease, disorder, or condition described herein. For example, in certain embodiments, administration of ISIS 681257 to a subject will prevent one or more symptoms of a cardiovascular disorder, e.g. administration of ISIS 681257 to a subject will inhibit or delay one or more symptoms associated with a cardiovascular disorder.

As used herein, "triglyceride" or "TG" means a lipid or neutral fat consisting of glycerol combined with three fatty acid molecules.

As used herein, "type 2 diabetes," (also known as "type 2 diabetes mellitus", "diabetes mellitus, type 2", "non-insulin-dependent diabetes", "NIDDM", "obesity related diabetes", or "adult-onset diabetes") is a metabolic disorder that is primarily characterized by insulin resistance, relative insulin deficiency, and hyperglycemia.

### Certain Embodiments

The present disclosure also provides for the use of the oligomeric compound ISIS 681257 in the manufacture of a medicament for treating, ameliorating, delaying or preventing one or more of a disease related to apo(a) and/or Lp(a), wherein from 75 mg to 85 mg of the oligomeric compound are administered to the human during a dosing period of one month.

ISIS 681257 has the following structure, which includes salts thereof:

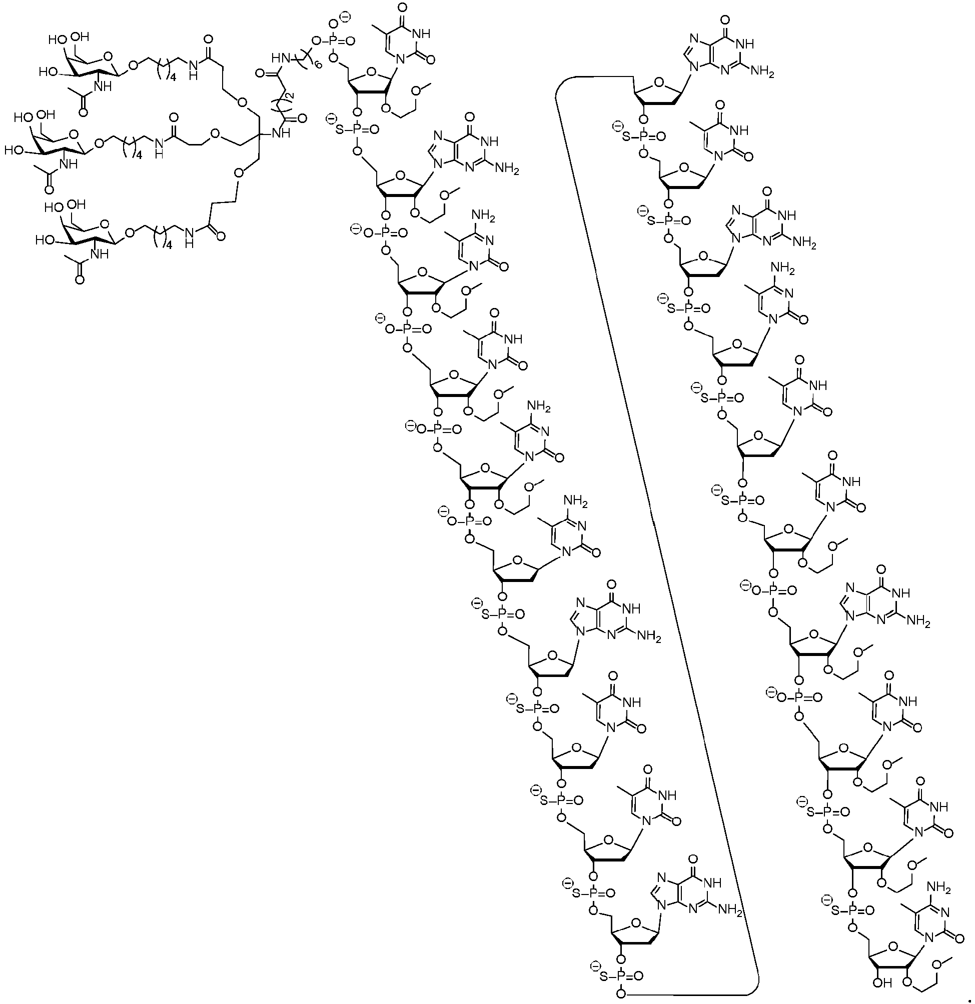

The present disclosure provides a kit comprising the oligomeric compound ISIS 681257 for use as claimed herein, wherein the kit comprises: (i) ISIS 681257; and optionally (ii) a second agent or therapy as described herein.

A kit of the present invention can further include instructions for using the kit to treat, prevent, or ameliorate a disease, disorder or condition as described herein by combination therapy as described herein.

### B. Apolipoprotein (a) (apo(a))

One apo(a) protein is linked via a disulfide bond to a single apolipoprotein B (apoB) protein to form a lipoprotein(a) (Lp(a)) particle. The apo(a) protein shares a high degree of homology with plasminogen particularly within the kringle IV type 2 repetitive domain. It is thought that the kringle repeat domain in apo(a) may be responsible for its pro-thrombotic and anti-fibrinolytic properties, potentially enhancing atherosclerotic progression. Apo(a) is transcriptionally regulated by IL-6 and in studies in rheumatoid arthritis patients treated with an IL-6 inhibitor (tocilizumab), plasma levels were reduced by 30% after 3 month treatment. Apo(a) has been shown to preferentially bind oxidized phospholipids and potentiate vascular inflammation. Further, studies suggest that the Lp(a) particle may also stimulate endothelial permeability, induce plasminogen activator inhibitor type-1 expression and activate macrophage interleukin-8 secretion. Importantly, recent genetic association studies revealed that Lp(a) was an independent risk factor for myocardial infarction, stroke, peripheral vascular disease and abdominal aortic aneurysm. Further, in the Precocious Coronary Artery Disease (PROCARDIS) study, Clarke *et al.* described robust and independent associations between coronary heart disease and plasma Lp(a) concentrations. Additionally, Solfrizzi et al., suggested that increased serum Lp(a) may be linked to an increased risk for Alzheimer's Disease (AD). Antisense compounds targeting apo(a) have been previously disclosed in WO2005/000201 and US2010-0331390 An antisense oligonucleobase targeting Apo(a), ISIS-APOA_{Rx}, was assessed in a Phase I clinical trial to study it's safety profile.

### Apo(a) Therapeutic Indications

The present disclosure provides ISIS 681257, which is a conjugated antisense compound targeted to an apo(a) nucleic acid for modulating the expression of apo(a) in a subject, for use as claimed herein. When administered to a human, ISIS 681257 reduces expression of apo(a).

In certain embodiments, the invention provides ISIS 681257 for use as claimed herein in a pharmaceutical composition for treating a subject. In certain embodiments, the individual has an apo(a) related disease. In certain embodiments, the individual has an Lp(a) related disease. In certain embodiments, the individual has an inflammatory, cardiovascular and/or a metabolic disease, disorder or condition. In certain embodiments, the subject has an inflammatory, cardiovascular and/or metabolic disease, disorder or condition.

In certain embodiments, the cardiovascular diseases, disorders or conditions (CVD) include, but are not limited to, elevated Lp(a) associated CVD risk, recurrent cardiovascular events with elevated Lp(a), aortic stenosis (e.g., calcific aortic valve stenosis associated with high Lp(a)), aneurysm (e.g., abdominal aortic aneurysm), angina, arrhythmia, atherosclerosis, cerebrovascular disease, coronary artery disease, coronary heart disease, dyslipidemia, hypercholesterolemia, hyperlipidemia, hypertension, hypertriglyceridemia, myocardial infarction, peripheral vascular disease (e.g., peripheral artery disease), stroke and the like.

In certain embodiments, ISIS 681257 modulates physiological markers or phenotypes of the cardiovascular disease, disorder or condition. For example, administration of ISIS 681257 to a human can decrease Lp(a), LDL and cholesterol levels compared to untreated subjects. In certain embodiments, the modulation of the physiological markers or phenotypes can be associated with inhibition of apo(a) by ISIS 681257.

In certain embodiments, the physiological markers of the cardiovascular disease, disorder or condition can be quantifiable. For example, Lp(a), LDL or cholesterol levels can be measured and quantified by, for example, standard lipid tests. For such markers, in certain embodiments, the marker can be decreased by about 5, 10, 15, 20, 25, 30, 35, 40, 45, 50, 55, 60, 65, 70, 75, 80, 85, 90, 95 or 99%, or a range defined by any two of these values.

Also, provided herein is the compound for use as claimed herein for preventing, treating or ameliorating a symptom associated with the cardiovascular disease, disorder or condition in a subject in need thereof. In certain embodiments, provided is the compound for use as claimed herein for reducing the rate of onset of a symptom associated with the cardiovascular disease, disorder or condition. In certain embodiments, provided is the compound for use as claimed herein for reducing the severity of a symptom associated with the cardiovascular disease, disorder or condition. In such embodiments, the use comprise administering a therapeutically effective amount of ISIS 681257 to an individual in need thereof.

The cardiovascular disease, disorder or condition can be characterized by numerous physical symptoms. Any symptom known to one of skill in the art to be associated with the cardiovascular disease, disorder or condition can be prevented, treated, ameliorated or otherwise modulated with the compounds and uses described herein. In certain embodiments, the symptom can be any of, but not limited to, angina, chest pain, shortness of breath, palpitations, weakness, dizziness, nausea, sweating, tachycardia, bradycardia, arrhythmia, atrial fibrillation, swelling in the lower extremities, cyanosis, fatigue, fainting, numbness of the face, numbness of the limbs, claudication or cramping of muscles, bloating of the abdomen or fever.

In certain embodiments, the metabolic diseases, disorders or conditions include, but are not limited to, hyperglycemia, prediabetes, diabetes (type I and type II), obesity, insulin resistance, metabolic syndrome and diabetic dyslipidemia.

In certain embodiments, ISIS 681257 modulates physiological markers or phenotypes of the metabolic disease, disorder or condition. For example, administration of ISIS 681257 to humans can decrease glucose and insulin resistance levels in those subjects compared to untreated subjects. In certain embodiments, the modulation of the physiological markers or phenotypes can be associated with inhibition of apo(a) by ISIS 681257.

In certain embodiments, physiological markers of the metabolic disease, disorder or condition can be quantifiable. For example, glucose levels or insulin resistance can be measured and quantified by standard tests known in the art. For such markers, in certain embodiments, the marker can be decreased by about 5, 10, 15, 20, 25, 30, 35, 40, 45, 50, 55, 60, 65, 70, 75, 80, 85, 90, 95 or 99%, or a range defined by any two of these values. In another example, insulin sensitivity can be measured and quantified by standard tests known in the art. For such markers, in certain embodiments, the marker can be increase by about 5, 10, 15, 20, 25, 30, 35, 40, 45, 50, 55, 60, 65, 70, 75, 80, 85, 90, 95 or 99%, or a range defined by any two of these values.

Also, provided herein is the compound for use as claimed herein for preventing, treating or ameliorating a symptom associated with the metabolic disease, disorder or condition in a subject in need thereof. In certain embodiments, provided is the compound for use as claimed herein for reducing the rate of onset of a symptom associated with the metabolic disease, disorder or condition. In certain embodiments, provided is the compound for use as claimed herein for reducing the severity of a symptom associated with the metabolic disease, disorder or condition. In such embodiments, the uses comprise administering a therapeutically effective amount of ISIS 681257 to an individual in need thereof.

The metabolic disease, disorder or condition can be characterized by numerous physical symptoms. Any symptom known to one of skill in the art to be associated with the metabolic disease, disorder or condition can be prevented, treated, ameliorated or otherwise modulated with the compounds for use as described herein. In certain embodiments, the symptom can be any of, but not limited to, excessive urine production (polyuria), excessive thirst and increased fluid intake (polydipsia), blurred vision, unexplained weight loss and lethargy.

In certain embodiments, the inflammatory diseases, disorders or conditions include, but are not limited to, elevated Lp(a) associated CVD risk, recurrent cardiovascular events with elevated Lp(a), aortic stenosis (e.g., calcific aortic valve stenosis associated with high Lp(a)), coronary artey disease (CAD), Alzheimer's Disease and thromboembolic diseases, disorder or conditions. Certain thromboembolic diseases, disorders or conditions include, but are not limited to, stroke, thrombosis, myocardial infarction and peripheral vascular disease.

In certain embodiments, ISIS 681257 modulates physiological markers or phenotypes of the inflammatory disease, disorder or condition. For example, administration of ISIS 681257 to a human can decrease inflammatory cytokine or other inflammatory markers levels in compared to untreated subjects. In certain embodiments, the modulation of the physiological markers or phenotypes can be associated with inhibition of apo(a) by ISIS 681257.

In certain embodiments, the physiological markers of the inflammatory disease, disorder or condition can be quantifiable. For example, cytokine levels can be measured and quantified by standard tests known in the art. For such markers, in certain embodiments, the marker can be decreased by at least about 5%, 10%, 15%, 20%, 25%, 30%, 35%, 40%, 45%, 50%, 55%, 60%, 65%, 70%, 75%, 80%, 85%, 90%, 95% or 99%, or a range defined by any two of these values.

Also, provided herein is ISIS 681257 for use as claimed herein, wherein the use is for preventing, treating or ameliorating a symptom associated with the inflammatory disease, disorder or condition in a subject in need thereof. In certain embodiments, provided is ISIS 681257 for use as claimed herein, wherein the use is for reducing the rate of onset of a symptom associated with the inflammatory disease, disorder or condition. In certain embodiments, provided is ISIS 681257 for use as claimed herein, wherein the use is for reducing the severity of a symptom associated with the inflammatory disease, disorder or condition. In such embodiments, the uses comprise administering a therapeutically effective amount of ISIS 681257 to an individual in need thereof.

In certain embodiments, provided is ISIS 681257 for use as claimed herein, wherein the use is treating an individual with an apo(a) related disease, disorder or condition comprising administering a therapeutically effective amount of one or more pharmaceutical compositions as described herein. In certain embodiments, the individual has elevated apo(a) levels. In certain embodiments, provided ISIS 681257 for use as claimed herein, wherein the use is treating an individual with an Lp(a) related disease, disorder or condition comprising administering a therapeutically effective amount of one or more pharmaceutical compositions as described herein. In certain embodiments, the individual has elevated Lp(a) levels. In certain embodiments, the individual has an inflammatory, cardiovascular and/or metabolic disease, disorder or condition. In certain embodiments, administration of a therapeutically effective amount of ISIS 681257 is accompanied by monitoring of apo(a) or Lp(a) levels. In certain embodiments, administration of a therapeutically effective amount of ISIS 681257 is accompanied by monitoring of markers of inflammatory, cardiovascular and/or metabolic disease, or other disease process associated with the expression of apo(a), to determine an individual's response to ISIS 681257. An individual's response to administration of ISIS 681257 can be used by a physician to determine the amount and duration of therapeutic intervention with the ISIS 681257.

In certain embodiments, administration of ISIS 681257 results in reduction of apo(a) expression by at least about 15%, 20%, 25%, 30%, 35%, 40%, 45%, 50%, 55%, 60%, 65%, 70%, 75%, 80%, 85%, 90%, 95% or 99%, or a range defined by any two of these values. In certain embodiments, apo(a) expression is reduced to at least ≤ 100 mg/dL, ≤ 90 mg/dL, ≤ 80 mg/dL, ≤ 70 mg/dL, ≤ 60 mg/dL, ≤ 50 mg/dL, ≤ 40 mg/dL, ≤ 30 mg/dL, ≤20 mg/dL or ≤ 10 mg/dL.

In certain embodiments, administration of ISIS 681257 results in reduction of Lp(a) expression by at least about 15%, 20%, 25%, 30%, 35%, 40%, 45%, 50%, 55%, 60%, 65%, 70%, 75%, 80%, 85%, 90%, 95% or 99%, or a range defined by any two of these values. In certain embodiments, Lp(a) expression is reduced to at least ≤ 200 mg/dL, ≤ 190 mg/dL, ≤ 180 mg/dL, ≤ 175 mg/dL, ≤ 170 mg/dL, ≤ 160 mg/dL, ≤ 150 mg/dL, ≤ 140 mg/dL, ≤ 130 mg/dL, ≤ 120 mg/dL, ≤ 110 mg/dL, ≤ 100 mg/dL, ≤ 90 mg/dL, ≤ 80 mg/dL, ≤ 70 mg/dL, ≤ 60 mg/dL, ≤ 55 mg/dL, ≤ 50 mg/dL, ≤ 45 mg/dL, ≤ 40 mg/dL, ≤ 35 mg/dL, ≤ 30 mg/dL, ≤ 25 mg/dL, ≤ 20 mg/dL, ≤ 15 mg/dL, or ≤ 10 mg/dL.

In certain embodiments, the invention provides methods for using ISIS 681257 in the preparation of a medicament. In certain embodiments, pharmaceutical compositions comprising ISIS 681257 are used for the preparation of a medicament for treating a patient suffering or susceptible to an inflammatory, cardiovascular and/or a metabolic disease, disorder or condition.

### Apo(a) Treatment Populations

Certain subjects with high Lp(a) levels are at a significant risk of various diseases (Lippi et al., Clinica Chimica Acta, 2011, 412:797-801; Solfrizz et al.). For example, subjects will Lp(a) levels greater than ≥ 75 nanomoles/liter (nmol/L) or ≥ 30 mg/dL are considered to have increased risk for various diseases. In many subjects with high Lp(a) levels, current treatments cannot reduce their Lp(a) levels to safe levels. Apo(a) plays an important role in the formation of Lp(a), hence reducing apo(a) can reduce Lp(a) and prevent, treat or ameliorate a disease associated with Lp(a).

In certain embodiments, treatment with the compounds and uses disclosed herein is indicated for a human with elevated apo(a) levels and/or Lp(a) levels. In certain embodiments, the human has apo(a) levels ≥ 10 mg/dL, ≥ 20 mg/dL, ≥ 30 mg/dL, ≥ 40 mg/dL, ≥ 50 mg/dL, ≥ 60 mg/dL, ≥ 70 mg/dL, ≥ 80 mg/dL, ≥ 90 mg/dL or ≥ 100 mg/dL. In certain embodiments, the human has Lp(a) levels ≥ 10 mg/dL, ≥ 15 mg/dL, ≥ 20 mg/dL, ≥ 25 mg/dL, ≥ 30 mg/dL, ≥ 35 mg/dL, ≥ 40 mg/dL, ≥ 50 mg/dL, ≥ 60 mg/dL, ≥ 70 mg/dL, ≥ 80 mg/dL, ≥ 90 mg/dL, ≥ 100 mg/dL, ≥ 110 mg/dL, ≥ 120 mg/dL, ≥ 130 mg/dL, ≥ 140 mg/dL, ≥ 150 mg/dL, ≥ 160 mg/dL, ≥ 170 mg/dL, ≥ 175 mg/dL, ≥ 180 mg/dL, ≥ 190 mg/dL, ≥ 200 mg/dL.

In certain embodiments, the human has apo(a) levels greater than the upper limit of normal, e.g. wherein the human has apo(a) levels ≥ 30 mg/dL, ≥ 35 mg/dL, ≥ 40 mg/dL, ≥ 50 mg/dL, ≥ 60 mg/dL, ≥ 70 mg/dL, ≥ 80 mg/dL, ≥ 90 mg/dL, ≥ 100 mg/dL, ≥ 110 mg/dL, ≥ 120 mg/dL, ≥ 130 mg/dL, ≥ 140 mg/dL, ≥ 150 mg/dL, ≥ 160 mg/dL, ≥ 170 mg/dL, ≥ 175 mg/dL, ≥ 180 mg/dL, ≥ 190 mg/dL, ≥ 200 mg/dL.

### Certain Apo(a) Dosing Regmines

In certain embodiments, 80 mg of ISIS 681257 is administered to a human subject.

In certain embodiments, only one dose is given during the dosing period.

In certain embodiments, 20 mg of ISIS 681257 is administered to a human subject each week.

In certain embodiments, 80 mg of ISIS 681257 is administered to a human subject during a dosing period.

In certain embodiments, 80 mg of ISIS 681257 is administered to a human subject once every four weeks.

In certain embodiments, 80 mg of ISIS 681257 is administered to a human subject once every month.

### C. Certain Pharmaceutical Compositions

In certain embodiments, the present disclosure provides pharmaceutical compositions comprising one or more antisense compound. In certain embodiments, such pharmaceutical composition comprises a suitable pharmaceutically acceptable diluent or carrier. In certain embodiments, a pharmaceutical composition comprises a sterile saline solution and one or more antisense compound. In certain embodiments, such pharmaceutical composition consists of a sterile saline solution and one or more antisense compound. In certain embodiments, the sterile saline is pharmaceutical grade saline. In certain embodiments, a pharmaceutical composition comprises one or more antisense compound and sterile water. In certain embodiments, a pharmaceutical composition consists of one or more antisense compound and sterile water. In certain embodiments, the sterile saline is pharmaceutical grade water. In certain embodiments, a pharmaceutical composition comprises one or more antisense compound and phosphate-buffered saline (PBS). In certain embodiments, a pharmaceutical composition consists of one or more antisense compound and sterile phosphate-buffered saline (PBS). In certain embodiments, the sterile saline is pharmaceutical grade PBS.

In certain embodiments, antisense compounds may be admixed with pharmaceutically acceptable active and/or inert substances for the preparation of pharmaceutical compositions or formulations. Compositions and methods for the formulation of pharmaceutical compositions depend on a number of criteria, including, but not limited to, route of administration, extent of disease, or dose to be administered.

In certain embodiments, pharmaceutical compositions provided herein comprise one or more modified oligonucleotides and one or more excipients. In certain such embodiments, excipients are selected from water, salt solutions, alcohol, polyethylene glycols, gelatin, lactose, amylase, magnesium stearate, talc, silicic acid, viscous paraffin, hydroxymethylcellulose and polyvinylpyrrolidone.

In certain embodiments, a pharmaceutical composition provided herein comprises a delivery system. Examples of delivery systems include, but are not limited to, liposomes and emulsions. Certain delivery systems are useful for preparing certain pharmaceutical compositions including those comprising hydrophobic compounds. In certain embodiments, certain organic solvents such as dimethylsulfoxide are used.

In certain embodiments, a pharmaceutical composition is prepared for administration by injection (e.g., intravenous, subcutaneous, intramuscular, etc.). In certain of such embodiments, a pharmaceutical composition comprises a carrier and is formulated in aqueous solution, such as water or physiologically compatible buffers such as Hanks's solution, Ringer's solution, or physiological saline buffer. In certain embodiments, other ingredients are included (e.g., ingredients that aid in solubility or serve as preservatives). In certain embodiments, injectable suspensions are prepared using appropriate liquid carriers, suspending agents and the like. Certain pharmaceutical compositions for injection are presented in unit dosage form, e.g., in ampoules or in multi-dose containers. Certain pharmaceutical compositions for injection are suspensions, solutions or emulsions in oily or aqueous vehicles, and may contain formulatory agents such as suspending, stabilizing and/or dispersing agents. Certain solvents suitable for use in pharmaceutical compositions for injection include, but are not limited to, lipophilic solvents and fatty oils, such as sesame oil, synthetic fatty acid esters, such as ethyl oleate or triglycerides, and liposomes. Aqueous injection suspensions may contain substances that increase the viscosity of the suspension, such as sodium carboxymethyl cellulose, sorbitol, or dextran. Optionally, such suspensions may also contain suitable stabilizers or agents that increase the solubility of the pharmaceutical agents to allow for the preparation of highly concentrated solutions.

In certain embodiments, the present disclosure provides methods of administering a pharmaceutical composition comprising an oligonucleotide of the present disclosure to a subject. Suitable administration routes include parenteral (e.g., intravenous, intramuscular, intramedullary, and subcutaneous).

### Nonlimiting disclosure

While certain compounds, compositions and methods described herein have been described with specificity in accordance with certain embodiments, the following examples serve only to illustrate the compounds described herein and are not intended to limit the same.

Although the sequence listing accompanying this filing identifies each sequence as either "RNA" or "DNA" as required, in reality, those sequences may be modified with any combination of chemical modifications. One of skill in the art will readily appreciate that such designation as "RNA" or "DNA" to describe modified oligonucleotides is, in certain instances, arbitrary. For example, an oligonucleotide comprising a nucleoside comprising a 2'-OH sugar moiety and a thymine base could be described as a DNA having a modified sugar (2'-OH for the natural 2'-H of DNA) or as an RNA having a modified base (thymine (methylated uracil) for natural uracil of RNA).

Accordingly, nucleic acid sequences provided herein, including, but not limited to those in the sequence listing, are intended to encompass nucleic acids containing any combination of natural or modified RNA and/or DNA, including, but not limited to such nucleic acids having modified nucleobases. By way of further example and without limitation, an oligonucleotide having the nucleobase sequence "ATCGATCG" encompasses any oligonucleotides having such nucleobase sequence, whether modified or unmodified, including, but not limited to, such compounds comprising RNA bases, such as those having sequence "AUCGAUCG" and those having some DNA bases and some RNA bases such as "AUCGATCG" and oligonucleotides having other modified bases, such as "AT^{me}CGAUCG," wherein ^{me}C indicates a cytosine base comprising a methyl group at the 5-position.

### EXAMPLES

The following examples illustrate certain embodiments of the present disclosure and are not limiting. Moreover, where specific embodiments are provided, the inventors have contemplated generic application of those specific embodiments. For example, disclosure of an oligonucleotide having a particular motif provides reasonable support for additional oligonucleotides having the same or similar motif. And, for example, where a particular high-affinity modification appears at a particular position, other high-affinity modifications at the same position are considered suitable, unless otherwise indicated.

Some aspects of the following Examples, especially dosages not adding up to a 75-85 mg monthly dosing schedule, are not according to the invention and are present for illustration purposes only.

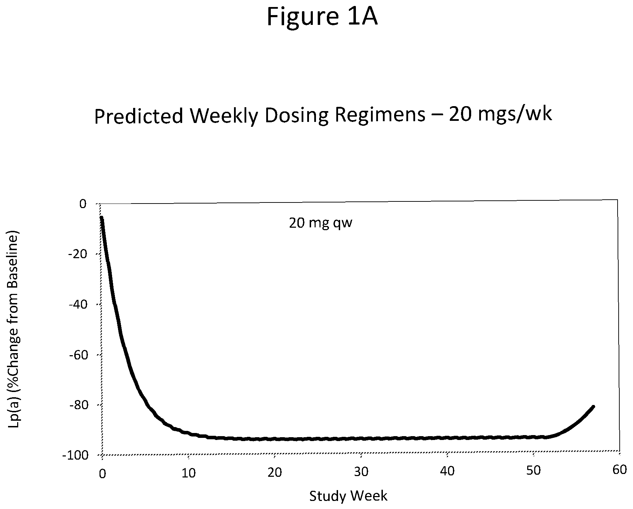

In particular, the Examples as depicted in Figures 1A-C, 2A, 3, 4, 5A-C, and 6A, 6B, and 6D are not according to the invention and are present for illustration purposes only.

### Example 1: ISIS 681257 Clinical Trial

As described herein, a double-blinded, placebo-controlled, dose-escalation Phase 1 study was performed on healthy volunteers with elevated Lp(a) to assess safety, tolerability, pharmacokinetics (PK) and pharmacodynamics (PD) after administration of single and multiple doses of ISIS 681257. ISIS 681257 was previously disclosed in WO 2014/179625 and is also described hereinabove. ISIS 681257 has been shown to be potent in inhibiting Lp(a) and tolerable when administered to non-human subjects. This subsequent study revealed unexpectedly improved properties of ISIS 681257 when administered to human subjects.

### Screening

Up to 28 days prior to treatment, subjects were screened for eligibility to participate in the study. Admission criteria for the study include the following:
1. Healthy males or females aged 18-65 inclusive and weighing ≥ 50 kg at the time of informed consent
2. BMI < 35.0 kg/m2
3. Subjects must have Lp(a) ≥ 75 nanomoles/liter (nmol/L) (≥ 30 mg/dL) at Screening. The Lp(a) value obtained via the Lp(a) pre-screening protocol may also be used to meet this criterion if measured within 6 months of dosing.

### Study Drug

Solutions of the Study Drug ISIS 681257 (100 mg/mL, 0.8 mL) contained in stoppered glass vials was used. Vials were for single use only. Doses of ISIS 681257 solution and placebo (0.9% sterile saline) were prepared by an unblinded pharmacist (or qualified delegate). A trained professional administered the ISIS 681257 or placebo blindly as a subcutaneous (sc) injection(s) in the abdomen, thigh, or outer area of the upper arm on each dosing day.

### Treatment and Post-Treatment Evaluation

Subjects enrolled in the study were split into 2 treatment arms: Single Ascending Dose (SAD) or Multiple Ascending Dose (MAD).

### Example 1A: Single Ascending Dose (SAD)

Approximately 28 subjects were enrolled in the SAD arm of this study, grouped into cohorts of 4 or 8 subjects randomized 3:1, ISIS 681257 to placebo. The subjects were administered placebo or ISIS 681257 at the doses listed in Table 1.

**Table 1: Single Ascending Doses**

| Cohort (n) | Dose |
|---|---|
| A (4) | 10 mg |
| B (4) | 20 mg |
| C (4) | 40 mg |
| D (8) | 80 mg |
| E (8) | 120 mg |

After treatment with a single dose of ISIS 681257 or placebo, the subjects were followed for up to 90 days to monitor the safety, tolerability, PK and PD of the drug. During the follow-up period, subjects return to the Study Center for visits on Study Days 2, 3, 8, 15 and 30 post-treatment (and Days 50, 70 and 90 post-treatment for Cohorts C, D and E) for safety and laboratory evaluations (blood draws), monitoring, concomitant medication usage recording, and adverse event (AE) collection. Collection of urine and feces was also performed on certain days. All visits by the subject for post-treatment assessment had a visit window of up to ± 1 days.

Analysis of serum samples showed dose dependent reductions in Lp(a) levels after a single dose of ISIS 681257 as measured 2 days, 4 days, 8 days, 15 days and 30 days post-treatment (Cohorts C, D and E were also assessed about 50 days, 70 days and 90 days post-treatment). Results, presented as a mean percent change in Lp(a) from baseline, are shown in Table 2.

**Table 2: Dose-dependent Change in Lp(a) after a Single Dose of ISIS 681257**

| | % Change from Baseline | | | | | | | |
|---|---|---|---|---|---|---|---|---|
| Cohort | Day 2 | Day 4 | Day 8 | Day 15 | Day 30 | Day 50 | Day 70 | Day 90 |
| Placebo | -1.7 | -2.4 | -9.6 | 0.3 | 6.8 | -14 | -9 | 3.9 |
| A | 4 | -5 | -16 | -20 | -26 | - | - | - |
| B | 7 | 8 | 2 | -22 | -33 | - | - | - |
| C | -2 | -13 | -33 | -41 | -43 | -35 | -26 | -26 |
| D | -21 | -35 | -50 | -70 | -79 | -71 | -52 | -46 |
| E | -11 | -25 | -50 | -76 | -85 | -75 | -61 | -44 |

Additionally, analysis of apo(a) isoforms, lipoprotein-associated phospholipase A2 (Lp-PLA2), secretory phospholipase A2 (sPLA2), oxidized phospholipid associated with apolipoprotein B (OxPL-apoB), and oxidized phospholipid associated with apolipoprotein(a) (OxPL-apo(a)) were performed.

During scheduled visits to the Study Center, the safety and tolerability of ISIS 681257 was clinically assessed in the subjects. Clinical staff assessed safety and tolerability by collecting and/or measuring one or more of the following: adverse events (AEs), quality of life assessments, concomitant medication/procedure information, vital signs, physical examination results (e.g., injection site reactions (ISRs) or flu-like symptoms (FLSs)), waist circumference, skinfold measurements, DEXA scans, electrocardiograms (ECGs), liver MRIs and echocardiograms.

Laboratory measurements such as serum chemistry (e.g., ALT, AST, bilirubin, creatinine, BUN), urinalysis, coagulation (e.g., aPTT (sec), PT (sec), INR, plasminogen), complement (e.g., C5a, Bb), hematology (e.g., hematocrit, white blood cells, platelets), immune function, thyroid function, inflammation (hsCRP), lipid panel (e.g., total cholesterol, HDL, LDL, TG, apoB, VLDL), ISIS 681257 plasma trough concentrations, and/or immunogenicity testing were performed on subject samples to assess the health and safety of each subject and the PD of the drug.

Laboratory measurements of subject samples were also used for PK profiling of the drug. For example, samples were used for measuring the amount and stability of ISIS 681257 and/or metabolites thereof, assessing drug binding proteins, and/or assessing other actions of ISIS 681257 with plasma constituents.

Both single dose treatment and multiple dose treatment with ISIS 681257 did not result in any safety or tolerability issues, at any of the clinically revelant doses tested. No ISRs were observed and no side effects were noted in any laboratory tests and the liver enzymes ALT and AST were not elevated.

The above results were surprising, because earlier experiments involving both the unconjugated compound (ISIS 494372) and the GalNAc conjugated compound (ISIS 681257) had suggested that the GalNAc conjugated compound would have significantly lower potency and/or a shorter duration of action in humans than was observed following the first dosing of humans reported herein (e.g. see Examples 89, 100 and 108 of WO 2014/179625 and Tsimikas et al., Lancet, 2015 Oct 10; 386:1472-83). In light of these surprising results, when treating humans, the GalNAc conjugated compound (ISIS 681257, or a salt thereof) can be administered at lower doses and/or less frequently than expected based on the earlier *in vivo* testing of the GalNAc conjugated compound. This can provide one or more very significant improvements in treating humans, e.g. reduced cost of treatment, improved patient compliance, reduced volume of administered medicinal product and/or potentially reduced risk of potential adverse events via lower dose administration regimens.

### Example 1B: Multiple Ascending Dose (MAD)

Thirty subjects were enrolled in the MAD arm of this study, grouped into cohorts of 10 randomized 4:1, ISIS 681257 to placebo. The subjects were administered a placebo or ISIS 681257 at the doses listed in Table 3. A total of 6 doses of drug or placebo was administered to each subject: loading doses were administered in the first week on Study Days 1 (first dose), 3, 5 and 8; maintenance doses were then administered weekly on Study Days 15 and 22.

**Table 3: Multiple Ascending Doses**

| Cohort (n) | ISIS 681257 Dose | # of Doses | Total Drug Dose |
|---|---|---|---|
| AA (10) | 10 mg | 6 | 60 mg |
| BB (10) | 20 mg | 6 | 120 mg |
| CC (10) | 40 mg | 6 | 240 mg |

During treatment with ISIS 681257 or placebo and for up to 13 weeks after treatment, the subjects were monitored for safety, tolerability, PK and PD of the drug. During the treatment period and the follow-up period, subjects return to the Study Center for visits on Study Days 5, 8, 15, 22, 29, 36, 50, 64, 85 and 113 for safety and clinical laboratory evaluations (blood draws), monitoring, concomitant medication usage recording, and AE event collection. Collection of urine and feces was also performed on certain days. All visits by the subject for post-treatment assessment had a visit window of up to ± 1 days.

Analysis of serum samples showed reductions in Lp(a) levels after multiple doses of ISIS 681257 as measured 5 days, 8 days, 15 days, 22 days, 29 days and 36 days after start of treatment. Results, presented as a mean percent change in Lp(a) from baseline, are shown in Table 4. Surprisingly, after a single dose of ISIS 681257, levels of Lp(a) continue to fall, reaching a nadir at about day 50 for the AA cohort. This demonstrates that the effective half life of ISIS 681257 appears to be much longer than anticipated. Additionally, cohorts BB and CC demonstrate continued reduction in Lp(a) through 36 days after administration of ISIS 681257.

**Table 4: Dose-dependent Reductions in Lp(a) after a Multiple Doses of ISIS-681257**

| | % Change from Baseline | | | | | | | | | |
|---|---|---|---|---|---|---|---|---|---|---|
| Cohort | Day 5 | Day 8 | Day 15 | Day 22 | Day 29 | Day 36 | Day 50 | Day 64 | Day 85 | Day 113 |
| Placebo | -2.6 | -11 | -11 | -4 | -3 | -10 | -1 | 18 | -1 | 10 |
| AA | -9 | -23 | -43 | -50 | -61 | -72 | -68 | -66 | -52 | -39 |
| BB | -10 | -20 | -52 | -68 | -75 | -80 | -80 | -77 | -64 | -52 |
| CC | -19 | -44 | -71 | -84 | -90 | -94 | -90 | -85 | -73 | -58 |

**Table 5: ED50 Values in Human**

| Regimen | ISIS 494372 | ISIS 681257 |
|---|---|---|
| Weekly Dose ED50 | 145 mg (1.5 ml) | 4.5 mg (0.05 ml) |

In human subjects, ISIS 681257 displayed dose-dependent, durable, statistically significant reductions in Lp(a) and an ED50 of 4.5 mg. ISIS 681257 was unexpectedly found to be ≥ 30-fold more potent than ISIS 494372 (an unconjugated antisense compound of the same nucleobase sequence and length; previously described in WO 2013/177468). Earlier experiments involving both ISIS 494372 and ISIS 681257 (reported in WO 2014/179625) had indicated that the GalNAc conjugated compound benefits from higher *in vivo* potency in mice, but these earlier experiments did not reveal or predict the unexpected ≥ 30-fold improvement in humans. Additionally, for the 10 mg multi-dose cohort, data points past Day 36 indicate that the nadir of Lp(a) levels for 6 of the 8 patients was not achieved until about Day 50, indicating that in humans ISIS 681257 showed an unexpected long half-life (T1/2) compared to ISIS 494372 (Tsimikas et al., Lancet, 2015 Oct 10; 386:1472-83) and this was likewise not revealed or predicted by earlier experiments in mice involving both ISIS 494372 and ISIS 681257.

Additionally, analysis of apo(a) isoforms, lipoprotein-associated phospholipase A2 (Lp-PLA2), secretory phospholipase A2 (sPLA2), oxidized phospholipid associated with apolipoprotein B (OxPL-apoB), and oxidized phospholipid associated with apolipoprotein(a) (OxPL-apo(a)) were performed. The results showed a significant reduction in LDL cholesterol, apolipoprotein B (apoB), and oxidised phospholipids (OxPL) associated with apoB and apo(a). Therefore, the reductions in Lp(a) occurred alongside significant reductions in proinflammatory OxPL, as well as reductions in LDL-C and apoB-100, which is consistent with a salutary effect on several causal pathways that mediate cardiovascular disease and calcific aortic valve stenosis. *See* Viney, et al. Lancet, 2016, Sep 2016; 388: 2239-53.

During scheduled visits to the Study Center, the safety and tolerability of ISIS 681257 was clinically assessed in the subjects. Clinical staff assessed safety and tolerability by collecting and/or measuring one or more of the following: adverse events (AEs), quality of life assessments, concomitant medication/procedure information, vital signs, physical examination results (e.g., injection site reactions (ISRs) or flu-like symptoms (FLSs)), waist circumference, skinfold measurements, DEXA scans, electrocardiograms (ECGs), liver MRIs and echocardiograms.

Laboratory measurements such as serum chemistry (e.g., ALT, AST, bilirubin, creatinine, BUN), urinalysis, coagulation (e.g., aPTT (sec), PT (sec), INR, plasminogen), complement (e.g., C5a, Bb), hematology (e.g., hematocrit, white blood cells, platelets), immune function, thyroid function, inflammation (hsCRP), lipid panel (e.g., total cholesterol, HDL, LDL, TG, apoB, VLDL), ISIS 681257 plasma trough concentrations, and/or immunogenicity testing were performed on subject samples to assess the health and safety of each subject and the PD of the drug.

Laboratory measurements of subject samples were also used for PK profiling of the drug. For example, samples were used for measuring the amount and stability of ISIS 681257 and/or metabolites thereof, assessing drug binding proteins, and/or assessing other actions of ISIS 681257 with plasma constituents.

Multiple dose treatments with ISIS 681257 did not result in any safety or tolerability issues. No ISR or FLS were observed. Liver enzymes ALT and AST were not elevated.

The ≥ 30-fold improvement in potency in humans was significantly greater than that expected. The above results were surprising, because earlier experiments involving both the unconjugated compound (ISIS 494372) and the GalNAc conjugated compound (ISIS 681257) had suggested that the GalNAc conjugated compound would have significantly lower potency and/or a shorter duration of action in humans than was observed following the first dosing of humans reported herein (e.g. see Examples 89, 100 and 108 of WO 2014/179625 and Tsimikas et al., Lancet, 2015 Oct 10; 386:1472-83). In light of these surprising results, when treating humans, the GalNAc conjugated compound (ISIS 681257, or a salt thereof) can be administered at lower doses and/or less frequently than expected based on the earlier *in vivo* testing of the GalNAc conjugated compound. This can provide one or more very significant improvements in treating humans, *e.g*. reduced cost of treatment, improved patient compliance, reduced volume of administered medicinal product and/or potentially reduced risk of potential adverse events via lower dose administration regimens.

### Example 2: Dose Regimens

Modeling based on the ongoing Phase 1clinical trial results was performed to assess optimal clinical dose regimens for ISIS 681257.

### Weekly Dosing

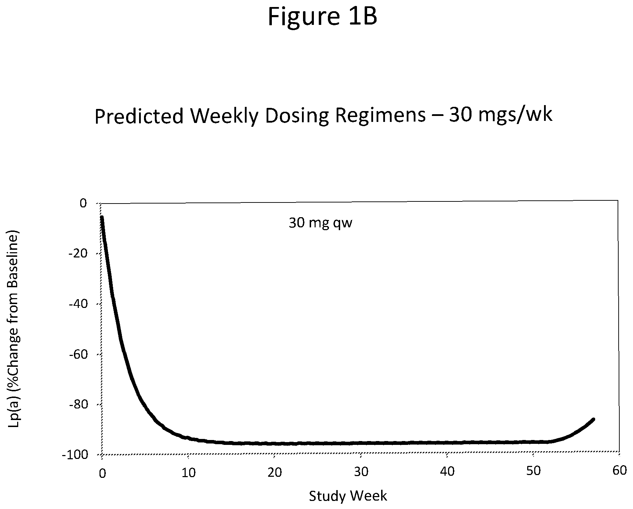

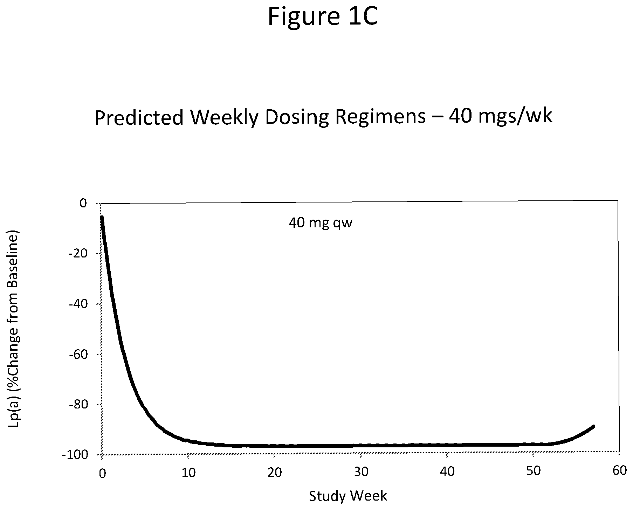

Figures 1A-C. Predicted Weekly Dosing Regimens. Charts are shown modeling the effect on Lp(a) by weekly administration of ISIS 681257 at doses of 20 mg (Fig 1A), 30 mg (Fig 1B) or 40 mg (Fig 1C). Lp(a) shows a steady state reduction of ≥ 80%.

### Monthly Dosing

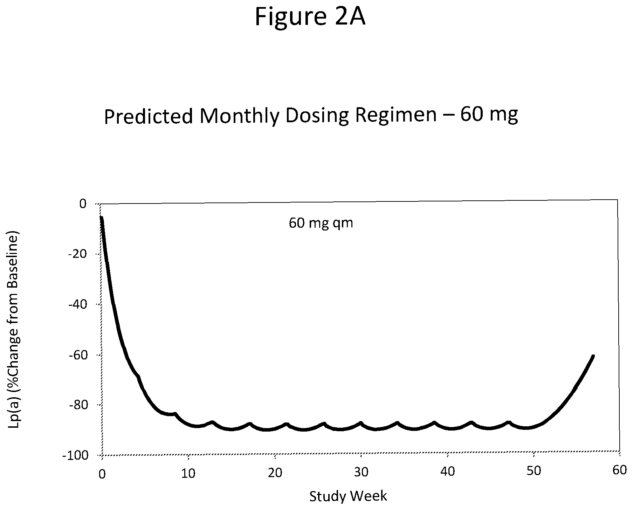

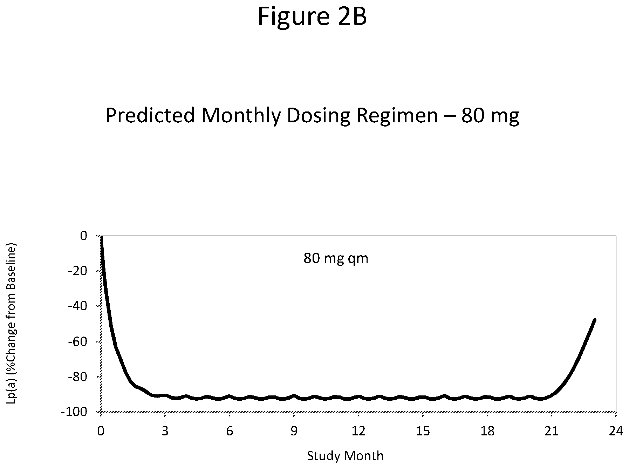

Figures 2A-B. Predicted Monthly Dosing Regimens. Chart are shown modeling the effect on Lp(a) by monthly administration of ISIS 681257 at dose of 60 mg (Fig 2A) and 80 mg (Fig 2B). Lp(a) shows a steady state reduction of about 80%.

### Two Months Dosing

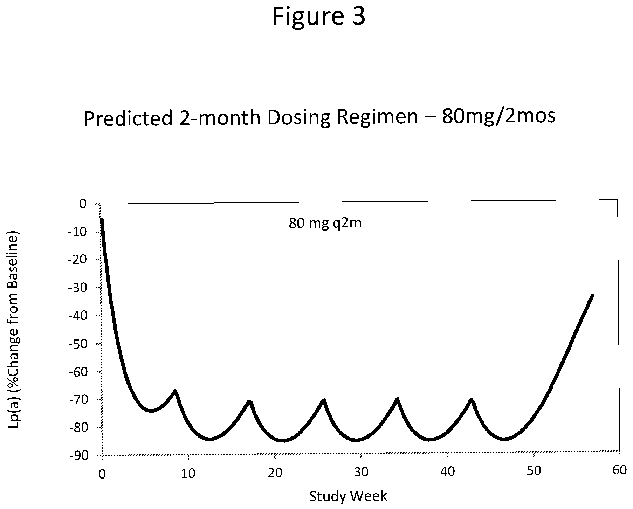

Figure 3. Predicted 2-month Dosing Regimen. A chart is shown modeling the effect on Lp(a) by administration of ISIS 681257 at an 80 mg dose every 2-months. Lp(a) shows a steady state reduction of about 80%.

### Quarterly dosing

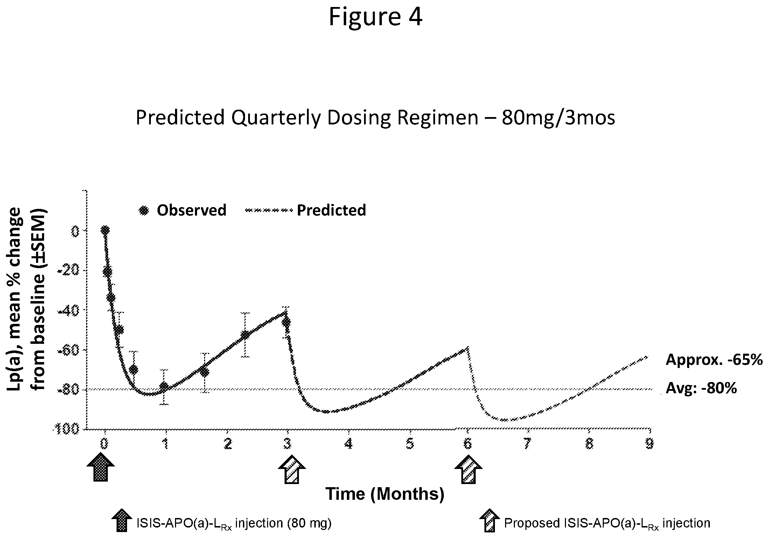

Figure 4. Predicted Quarterly Dosing Regimen. A chart is shown modeling the effect on Lp(a) by quarterly administration of ISIS 681257 at an 80 mg dose. Lp(a) shows a steady state reduction of 80% and maximum reduction of > 90%.

### Example 3: Dose Regimens

After completion of the phase 1 study described above, further modeling was performed to assess optimal clinical dose regimens for ISIS 681257.

### Weekly Dosing

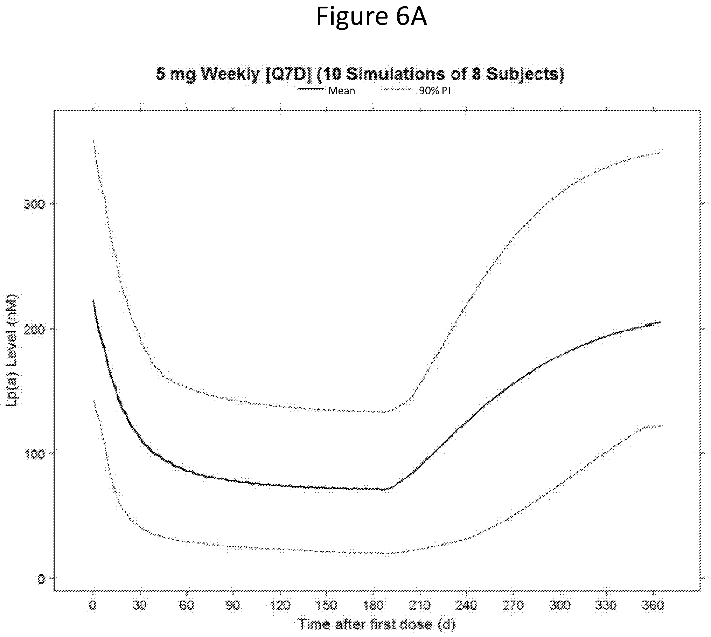

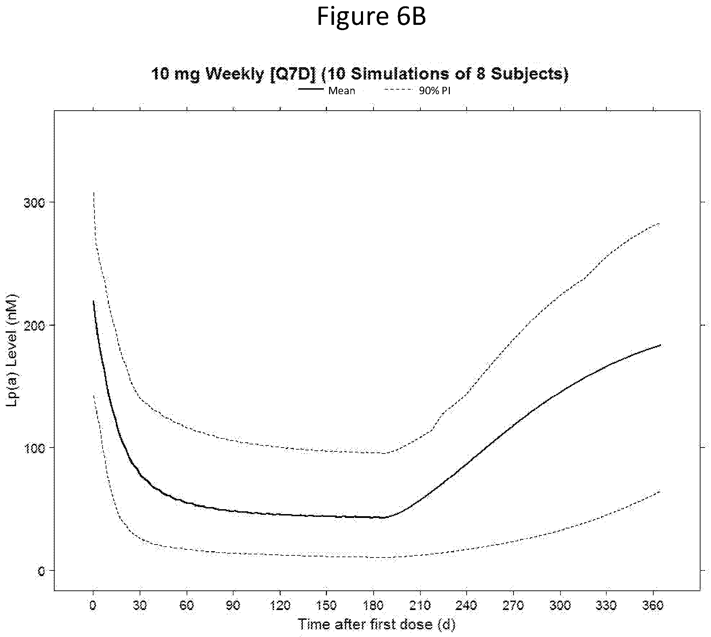

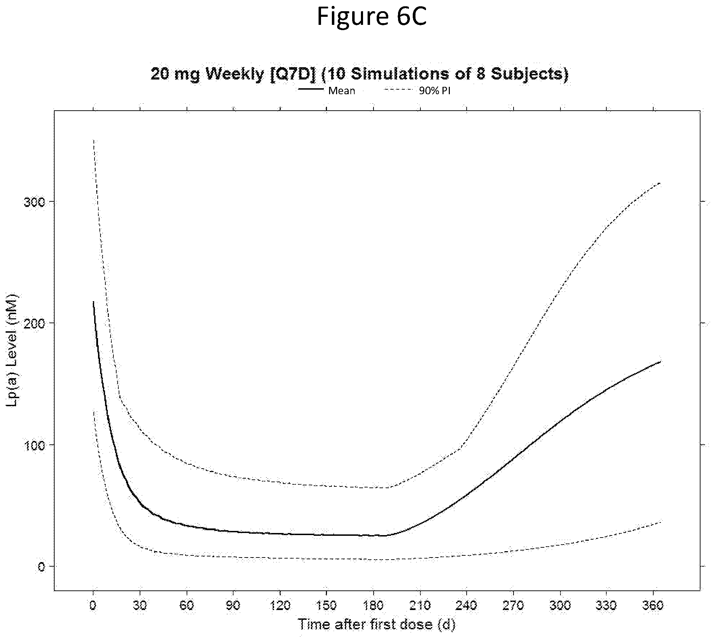

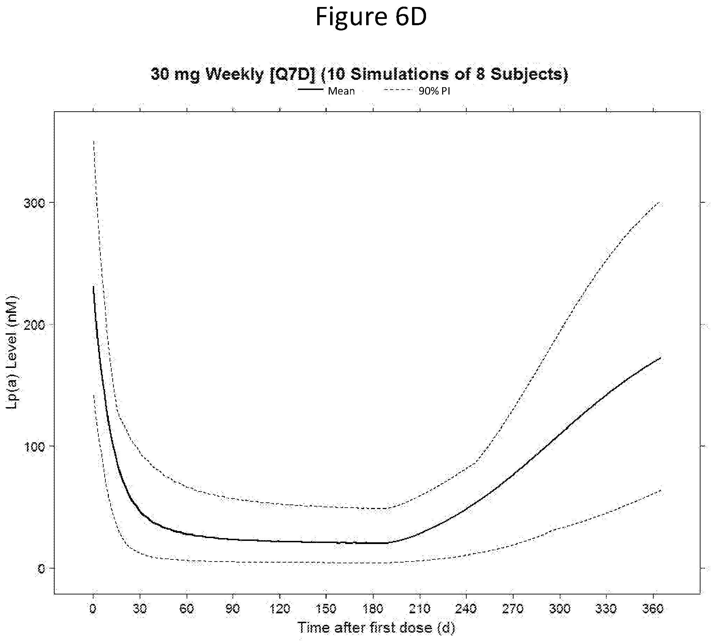

Figures 6A-D. Predicted Weekly Dosing Regimens. Charts are shown modeling the effect on Lp(a) by weekly administration of ISIS 681257 at doses of 5 mg (Fig 6A), 10 mg (Fig. 6B), 20 mg (Fig. 6C), and 30 mg (Fig. 6D). The dark middle line represents the predicted dose, while the uppermost and lowermost lines represent the 90% Confidence Interval.

### Monthly Dosing

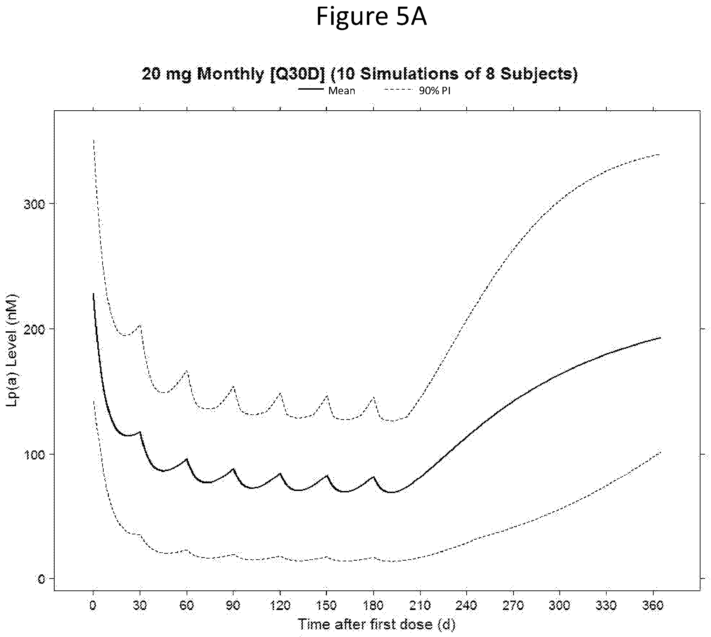

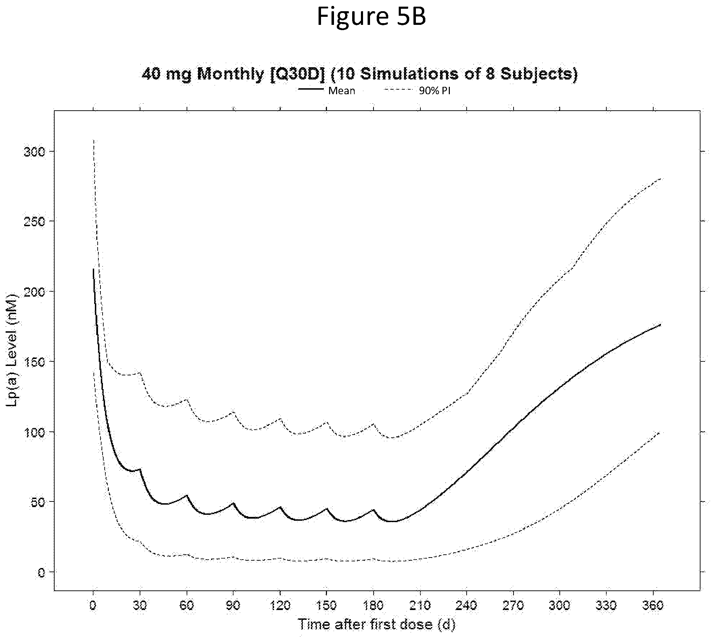

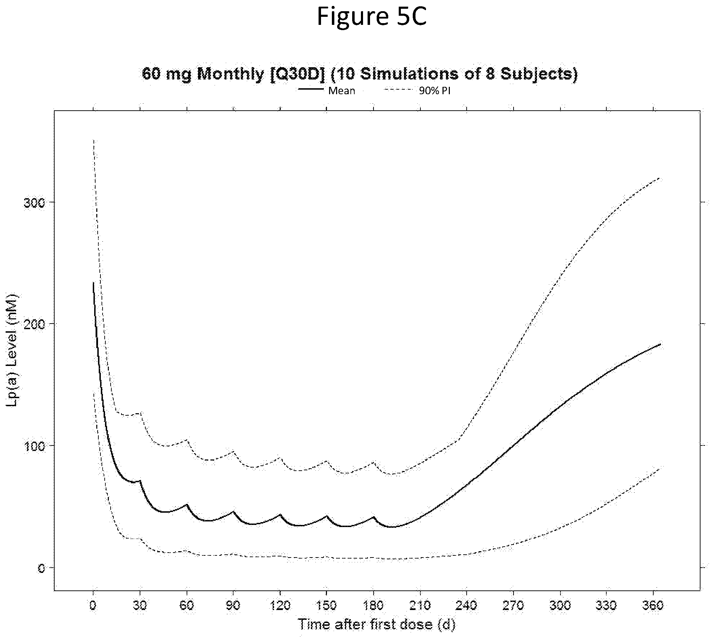

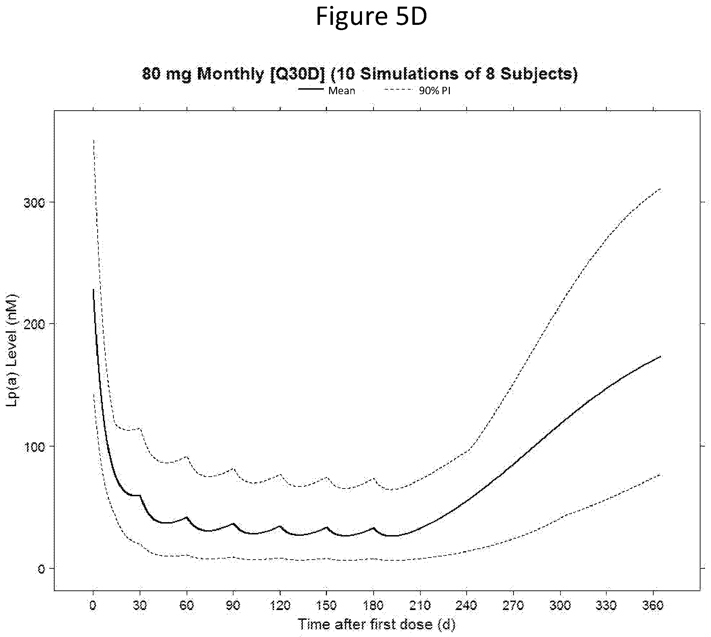

Figures 5A-D. Predicted Monthly Dosing Regimens. Charts are shown modeling the effect on Lp(a) by monthly administration of ISIS 681257 at doses of 20 mg (Fig 5A), 40 mg (Fig. 5B), 60 mg (Fig. 5C), and 80 mg (Fig.5D ). The dark middle line represents the predicted dose, while the uppermost and lowermost lines represent the 90% Confidence Interval.

### Example 4: A Randomized, Double-blind, Placebo-Controlled, Dose-Ranging Phase 2 Study of ISIS 681257 Administered Subcutaneously to Patients with Hyperlipoproteinemia(a) and Established Cardiovascular Disease (CVD)

The study described herein is to evaluate the safety, including tolerability, of ISIS 681257 and to assess the efficacy of different doses and dosing regimens of ISIS 681257 for reduction of plasma Lp(a) levels in patients with hyperlipoproteinemia(a) and established cardiovascular disease (CVD). CVD is defined as documented coronary artery disease, stroke, or peripheral artery disease. Patients must also have Lp(a) plasma level of ≥ 60 mg/dL. ISIS 681257 may provide therapeutic benefits to patients that have hyperlipoproteinemia(a) and established CVD.

Patient doses may be either 10 mg or 20 mg of ISIS 681257 administered once per week via subcutaneous injection for up to 52 weeks. Additional patient doses may be either 20mg, 40mg, or 60mg administered once every 4 weeks via subcutaneous injection for up to 13 administrations. The primary endpoint is the percent change in plasma Lp(a) from baseline at the primary analysis time point for ISIS 681257 treatment groups compared to placebo. The primary analysis time point is at Week 25 for patients who received every 4-week dosing and at Week 27 for patients who received weekly dosing. Secondary empoints may comprise the effect of ISIS 681257 as compared to placebo at the primary analysis time point on any one of the following:
- Percent change from baseline in LDL-C;
- Proportion of patients who achieve plasma Lp(a) ≤ 50 mg/dL;
- Proportion of patients who achieve plasma Lp(a) ≤ 30 mg/dL;
- Percent change from baseline in apoB;
- Percent change from baseline in OxPL-apo(a); and/or
- Percent change from baseline in OxPL-apoB.

This study may reveal unexpectedly improved properties of ISIS 681257 when administered to human subjects with hyperlipoproteinemia(a) and established cardiovascular disease (CVD). Treatment with ISIS 681257 may produce reduction in Lp(a) in patients with hyperlipoproteinemia(a) and established cardiovascular disease (CVD). Treatment with ISIS 681257 may produce reduction in baseline LDL-C, baseline apoB, baseline OxPL-apo(a), and/or baseline OxPL-apoB.

## Claims

1. An oligomeric compound having the following structure 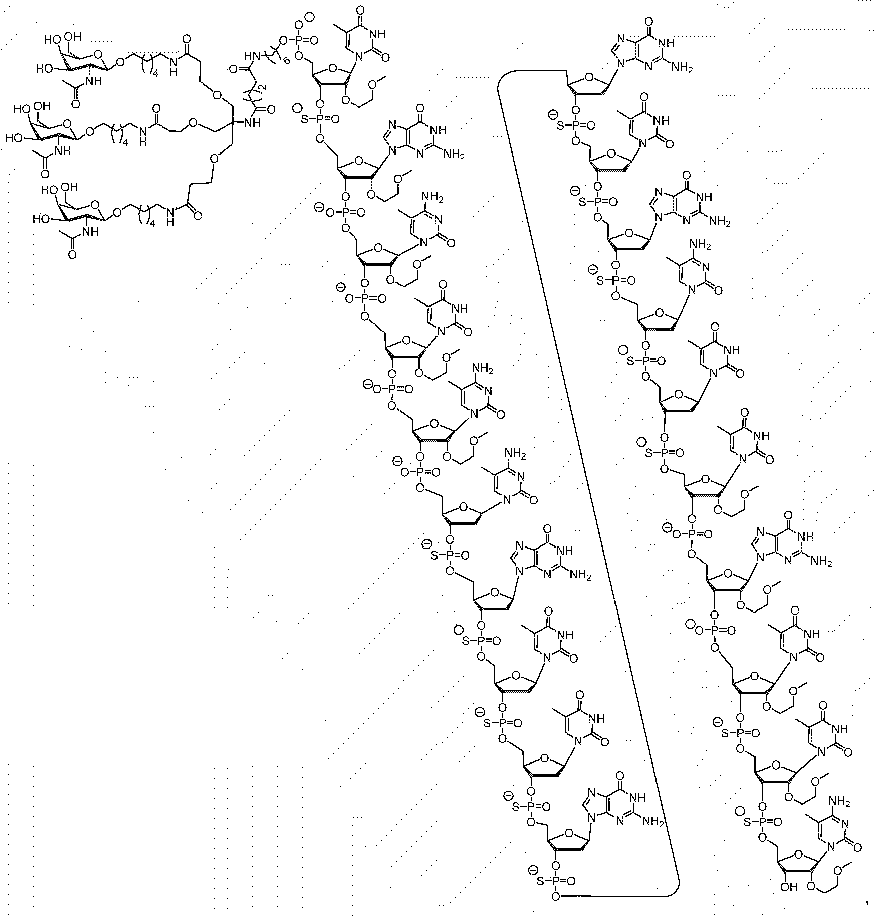 or any pharmaceutical acceptable salts thereof,
for use in treating or preventing a disease or condition in a human, wherein from 75 mg to 85 mg of the oligomeric compound are administered to the human during a dosing period of one month.

2. The oligomeric compound for use according to claim 1, wherein the 75 mg to 85 mg of the oligomeric compound are administered to the human in not more than one unit dose.

3. The oligomeric compound for use according to claim 2, wherein 80 mg of the oligomeric compound are administered to the human in not more than one unit dose.

4. The oligomeric compound for use according to claim 1, wherein 80 mg of the oligomeric compound are administered to the human during the dosing period.

5. An oligomeric compound having the following structure 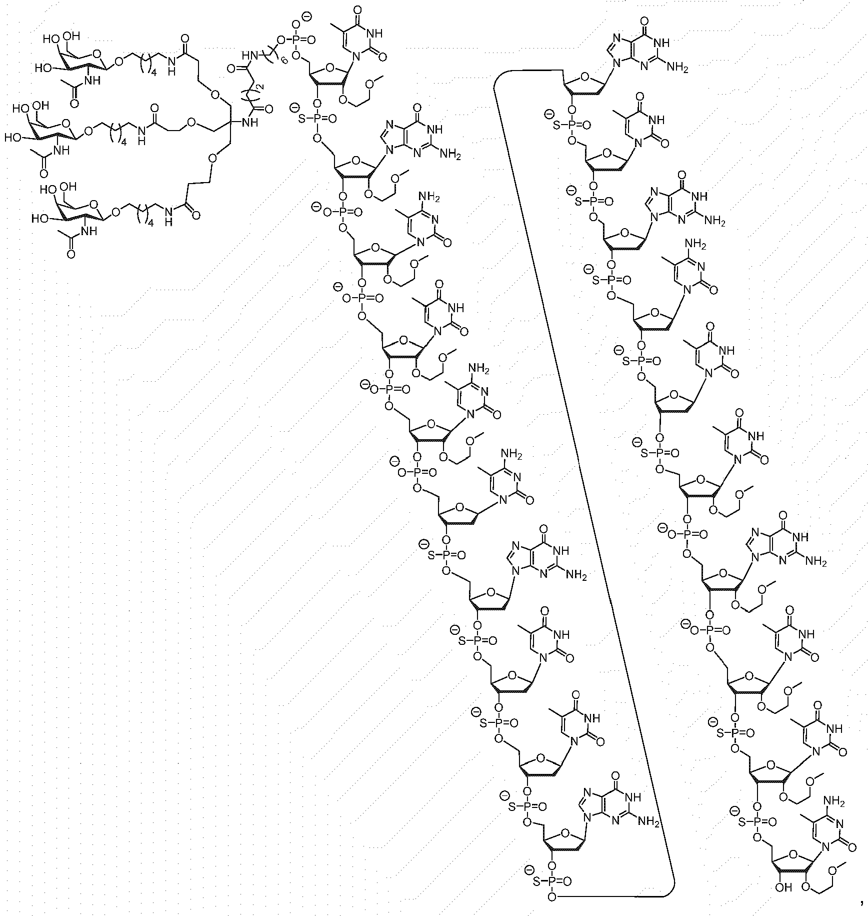 or any pharmaceutical acceptable salts thereof,
for use in treating or preventing a disease or condition in a human, wherein 80 mg of the oligomeric compound is administered to the human once every month.

6. The oligomeric compound for use according to any one of claims 1-5, wherein the oligomeric compound is administered as a sodium salt.

7. The oligomeric compound for use according to any one of claims 1-6, wherein the oligomeric compound has the following structure:

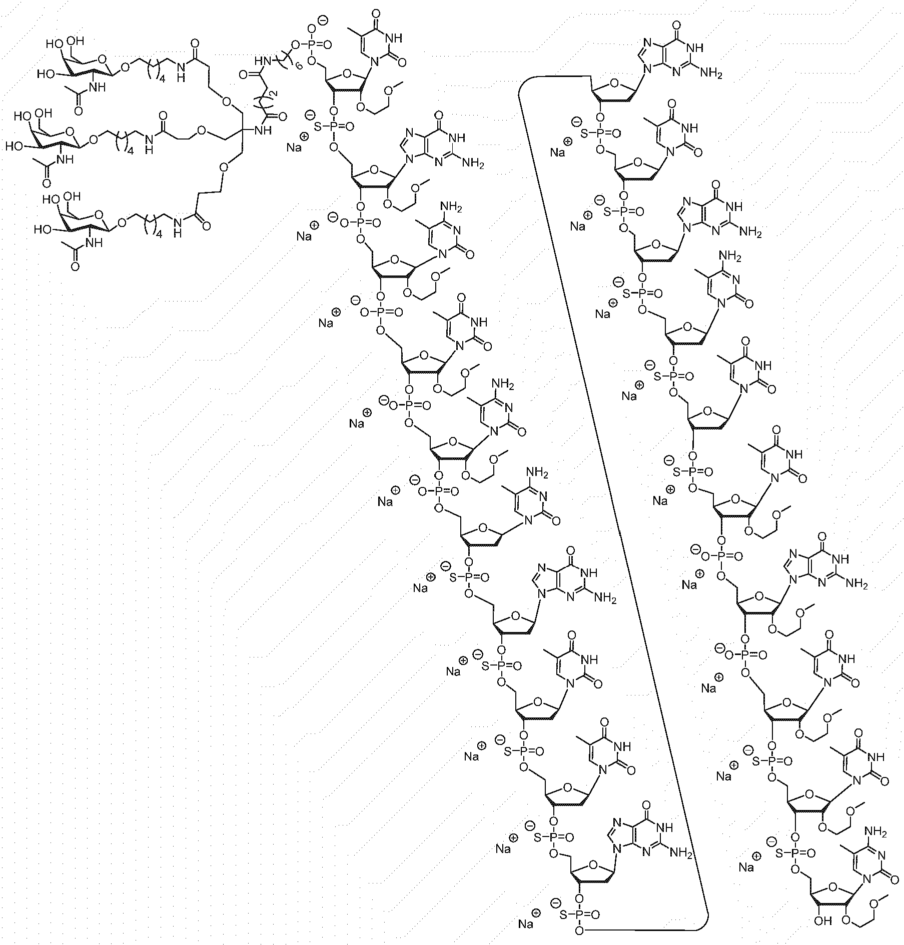

8. The oligomeric compound for use according to any one of claims 1-7, wherein the human is at enhanced risk for cardiovascular events due to chronically elevated plasma Lp(a) levels.

9. The oligomeric compound for use according to any one of claims 1-8, wherein the disease or condition is selected from calcific aortic valve stenosis with elevated Lp(a), elevated cardiovascular risk with elevated Lp(a), recurrent cardiovascular events with elevated Lp(a), or one or more symptoms of a cardiovascular disease or disorder associated with elevated Lp(a).

10. The oligomeric compound for use according to any one of claims 1-9, wherein the oligomeric compound is administered to the human by injection.

11. The oligomeric compound for use according to claim 10, wherein the oligomeric compound is administered to the human by subcutaneous injection.

12. The oligomeric compound for use according to any one of claims 1-11, wherein the oligomeric compound is formulated in a sterile liquid.

13. The oligomeric compound for use according to claim 12, wherein each unit dose of the oligomeric compound is not more than 1 mL, not more than 0.8 mL, not more than 0.5 mL, not more than 0.4 mL, not more than 0.25 mL, or not more than 0.2 mL of the sterile liquid.

14. The oligomeric compound for use according to claims 12 or 13, wherein:
(a) the sterile liquid is water,
(b) the sterile liquid is a physiologically compatible buffer,
(c) the sterile liquid is water with a sodium phosphate buffer, or
(d) the sterile liquid is water with a sodium phosphate buffer and sodium chloride.

15. The oligomeric compound for use according to any one of claims 12-14,
wherein the oligomeric compound is present at a concentration of 100 mg/mL in a 0.8 mL solution.

16. The oligomeric compound for use according to any one of claims 1-15, wherein administering the compound to a human reduces the fasting plasma Lp(a) concentration in the human by at least 50%, by at least 75%, by at least 80%, or by at least 85%, when the fasting plasma Lp(a) concentration in the human is measured at the start and end of the dosing period.

## Patentansprüche

1. Oligomere Verbindung mit der folgenden Struktur 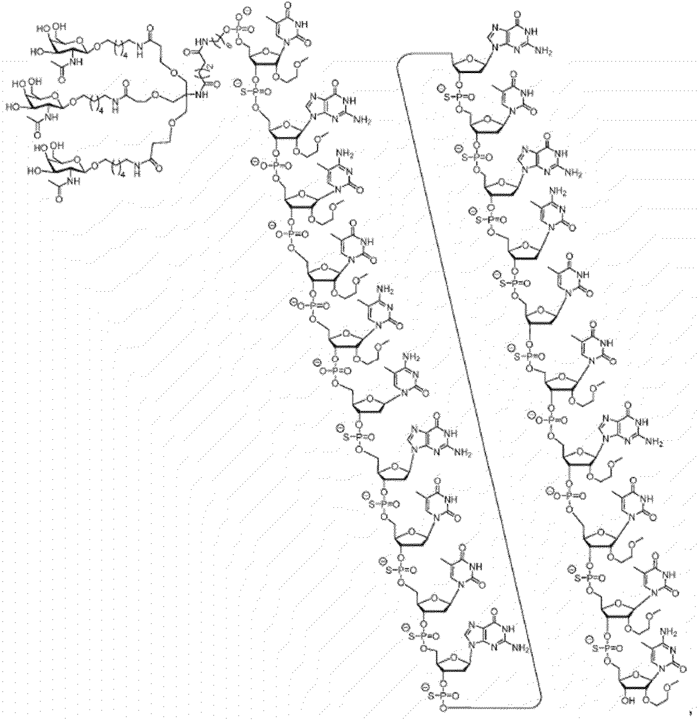
oder pharmazeutisch akzeptable Salze davon,
zur Verwendung bei der Behandlung oder Prävention einer Erkrankung oder eines Leidens bei einem Menschen, wobei 75 mg bis 85 mg der oligomeren Verbindung dem Menschen während eines Dosierungszeitraums von einem Monat verabreicht werden.

2. Oligomere Verbindung zur Verwendung nach Anspruch 1, wobei die 75 mg bis 85 mg der oligomeren Verbindung dem Menschen in nicht mehr als einer Einheitsdosis verabreicht werden.

3. Oligomere Verbindung zur Verwendung nach Anspruch 2, wobei dem Menschen 80 mg der oligomeren Verbindung in nicht mehr als einer Einheitsdosis verabreicht werden.

4. Oligomere Verbindung zur Verwendung nach Anspruch 1, wobei 80 mg der oligomeren Verbindung dem Menschen während des Dosierungszeitraums verabreicht werden.

5. Oligomere Verbindung mit der folgenden Struktur 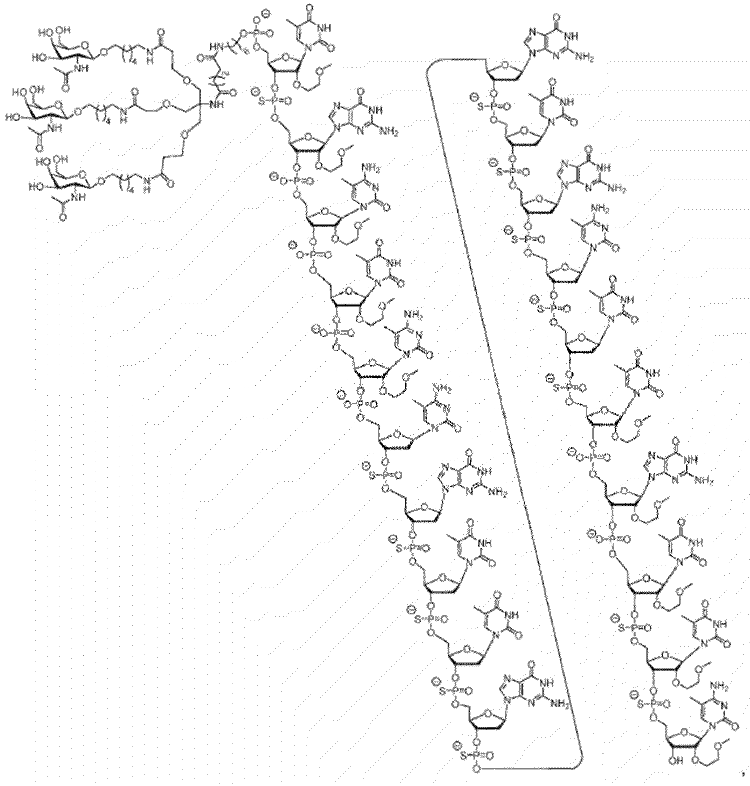
oder pharmazeutisch akzeptable Salze davon,
zur Verwendung bei der Behandlung oder Prävention einer Erkrankung oder eines Leidens bei einem Menschen, wobei dem Menschen 80 mg der oligomeren Verbindung einmal jeden Monat verabreicht werden.

6. Oligomere Verbindung zur Verwendung nach einem der Ansprüche 1-5, wobei die oligomere Verbindung als Natriumsalz verabreicht wird.

7. Oligomere Verbindung zur Verwendung nach einem der Ansprüche 1-6, wobei die oligomere Verbindung die folgende Struktur aufweist:

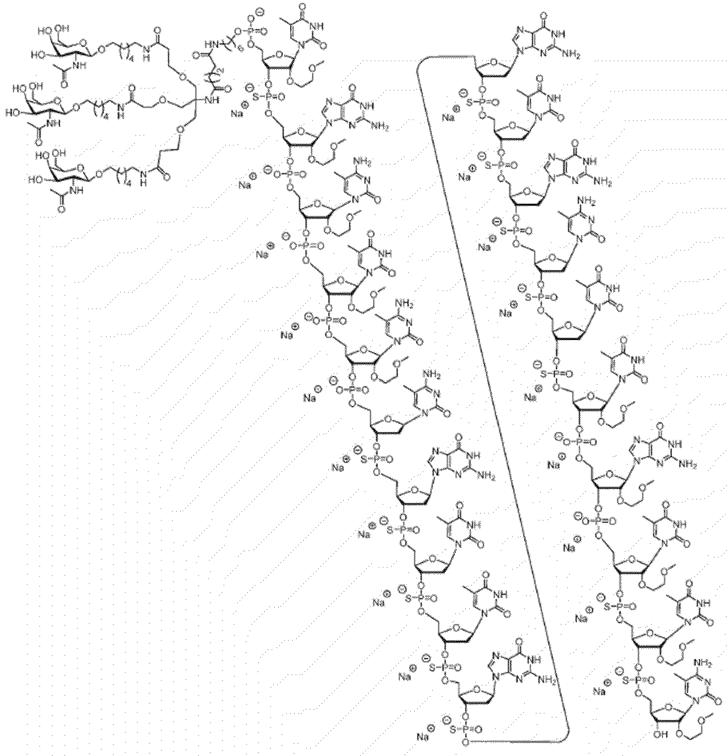

8. Oligomere Verbindung zur Verwendung nach einem der Ansprüche 1-7, wobei der Mensch aufgrund chronisch erhöhter Plasma-Lp(a)-Spiegel einem erhöhten Risiko für kardiovaskuläre Ereignisse ausgesetzt ist.

9. Oligomere Verbindung zur Verwendung nach einem der Ansprüche 1-8, wobei die Erkrankung oder das Leiden ausgewählt ist aus kalzifizierter Aortenklappenstenose mit erhöhtem Lp(a), erhöhtem kardiovaskulärem Risiko mit erhöhtem Lp(a), wiederkehrenden kardiovaskulären Ereignissen mit erhöhtem Lp(a) oder einem oder mehreren Symptomen einer kardiovaskulären Erkrankung oder Störung, die mit erhöhtem Lp(a) assoziiert ist.

10. Oligomere Verbindung zur Verwendung nach einem der Ansprüche 1-9, wobei die oligomere Verbindung dem Menschen durch Injektion verabreicht wird.

11. Oligomere Verbindung zur Verwendung nach Anspruch 10, wobei die oligomere Verbindung dem Menschen durch subkutane Injektion verabreicht wird.

12. Oligomere Verbindung zur Verwendung nach einem der Ansprüche 1-11, wobei die oligomere Verbindung in einer sterilen Flüssigkeit formuliert ist.

13. Oligomere Verbindung zur Verwendung nach Anspruch 12, wobei es sich bei jeder Einheitsdosis der oligomeren Verbindung um nicht mehr als 1 ml, nicht mehr als 0,8 ml, nicht mehr als 0,5 ml, nicht mehr als 0,4 ml, nicht mehr als 0,25 ml oder nicht mehr als 0,2 ml der sterilen Flüssigkeit handelt.

14. Oligomere Verbindung zur Verwendung nach Anspruch 12 oder 13, wobei:
(a) es sich bei der sterilen Flüssigkeit um Wasser handelt,
(b) es sich bei der sterilen Flüssigkeit um einen physiologisch verträglichen Puffer handelt,
(c) es sich bei der sterilen Flüssigkeit um Wasser mit einem Natriumphosphatpuffer handelt, oder
(d) es sich bei der sterilen Flüssigkeit um Wasser mit einem Natriumphosphatpuffer und Natriumchlorid handelt.

15. Oligomere Verbindung zur Verwendung nach einem der Ansprüche 12-14, wobei die oligomere Verbindung in einer Konzentration von 100 mg/ml in einer 0,8-ml-Lösung vorliegt.

16. Oligomere Verbindung zur Verwendung nach einem der Ansprüche 1-15, wobei das Verabreichen der Verbindung an einen Menschen die Nüchtern-Plasma-Lp(a)-Konzentration in dem Menschen um mindestens 50 %, um mindestens 75 %, um mindestens 80 % oder um mindestens 85 % reduziert, wenn die Nüchtern-Plasma-Lp(a)-Konzentration in dem Menschen zu Beginn und am Ende des Dosierungszeitraums gemessen wird.

## Revendications

1. Composé oligomère ayant la structure suivante 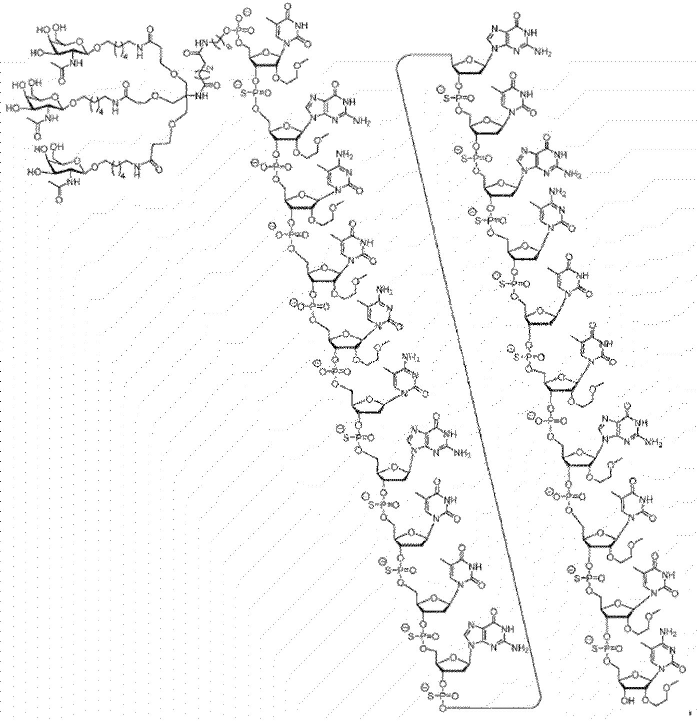
ou de quelconques sels pharmaceutiquement acceptables de celui-ci,
destiné à être utilisé en traitement ou prévention d'une maladie ou affection chez un être humain, de 75 mg à 85 mg du composé oligomère étant administrés à l'être humain pendant une période de traitement d'un mois.

2. Composé oligomère destiné à être utilisé selon la revendication 1, les 75 mg à 85 mg du composé oligomère étant administrés à l'être humain en pas plus d'une dose unitaire.

3. Composé oligomère destiné à être utilisé selon la revendication 2, 80 mg du composé oligomère étant administrés à l'être humain en pas plus d'une dose unitaire.

4. Composé oligomère destiné à être utilisé selon la revendication 1, 80 mg du composé oligomère étant administrés à l'être humain pendant la période de traitement.

5. Composé oligomère ayant la structure suivante 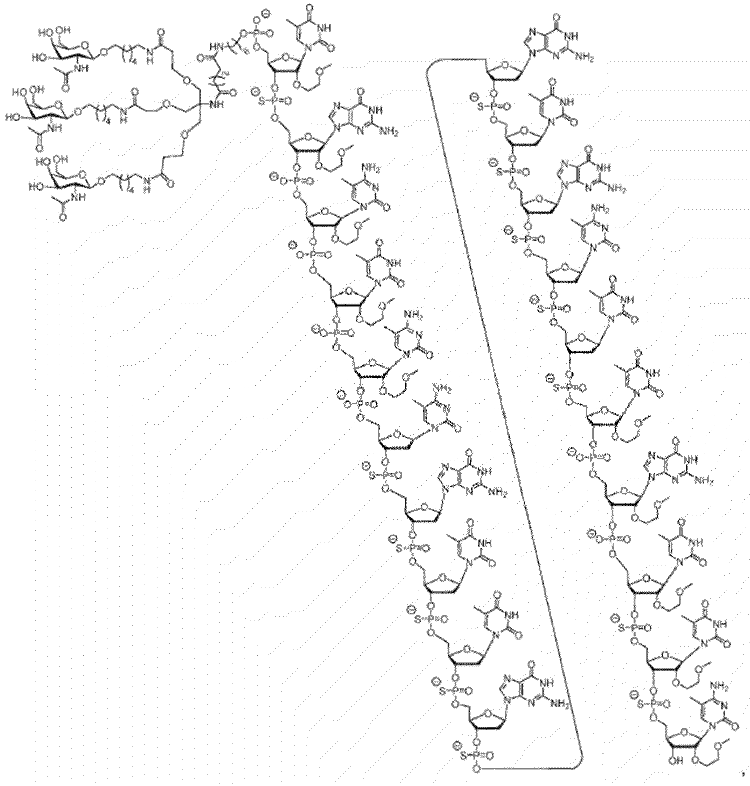
ou de quelconques sels pharmaceutiquement acceptables de celui-ci,
destiné à être utilisé en traitement ou prévention d'une maladie ou affection chez un être humain, 80 mg du composé oligomère étant administrés à l'être humain une fois par mois.

6. Composé oligomère destiné à être utilisé selon l'une quelconque des revendications 1 à 5, le composé oligomère étant administré sous forme d'un sel de sodium.

7. Composé oligomère destiné à être utilisé selon l'une quelconque des revendications 1 à 6, le composé oligomère ayant la structure suivante :

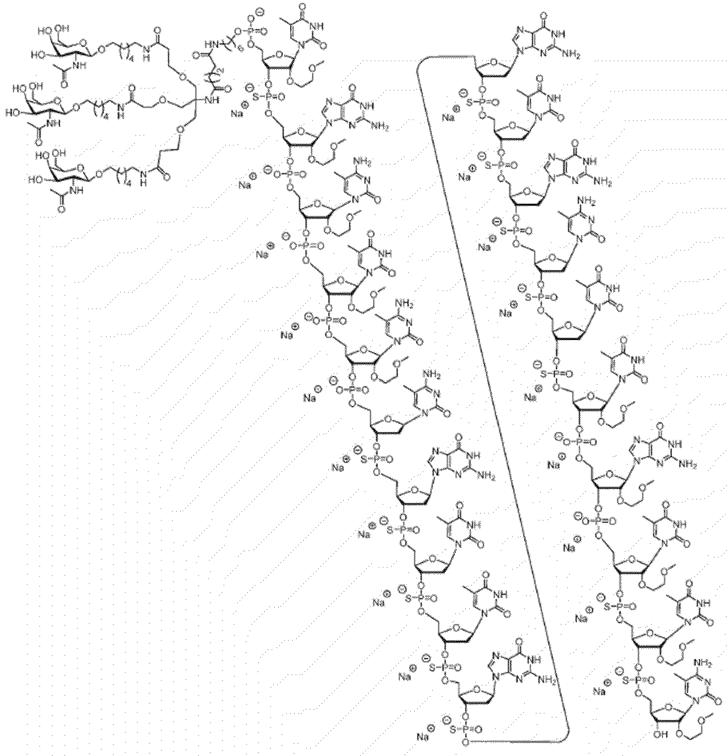

8. Composé oligomère destiné à être utilisé selon l'une quelconque des revendications 1 à 7, l'être humain présentant un risque accru d'événements cardiovasculaires en raison de taux plasmatiques de Lp(a) élevés de façon chronique.

9. Composé oligomère destiné à être utilisé selon l'une quelconque des revendications 1 à 8, la maladie ou affection étant choisie parmi une sténose aortique calcifiée avec un taux élevé de Lp(a), un risque cardiovasculaire élevé avec un taux élevé de Lp(a), des événements cardiovasculaires récurrents avec un taux élevé de Lp(a) ou un ou plusieurs symptômes d'une maladie ou d'un trouble cardiovasculaires associés à un taux élevé de Lp(a).

10. Composé oligomère destiné à être utilisé selon l'une quelconque des revendications 1 à 9, le composé oligomère étant administré à l'être humain par injection.

11. Composé oligomère destiné à être utilisé selon la revendication 10, le composé oligomère étant administré à l'être humain par injection sous-cutanée.

12. Composé oligomère destiné à être utilisé selon l'une quelconque des revendications 1 à 11, le composé oligomère étant formulé dans un liquide stérile.

13. Composé oligomère destiné à être utilisé selon la revendication 12, chaque dose unitaire du composé oligomère n'étant pas supérieure à 1 ml, pas supérieure à 0,8 ml, pas supérieure à 0,5 ml, pas supérieure à 0,4 ml, pas supérieure à 0,25 ml ou pas supérieure à 0,2 ml du liquide stérile.

14. Composé oligomère destiné à être utilisé selon les revendications 12 ou 13 :
(a) le liquide stérile étant de l'eau,
(b) le liquide stérile étant un tampon physiologiquement compatible,
(c) le liquide stérile étant de l'eau avec un tampon phosphate de sodium ou
(d) le liquide stérile étant de l'eau avec un tampon phosphate de sodium et du chlorure de sodium.

15. Composé oligomère destiné à être utilisé selon l'une quelconque des revendications 12 à 14, le composé oligomère étant présent en une concentration de 100 mg/ml dans une solution de 0,8 ml.

16. Composé oligomère destiné à être utilisé selon l'une quelconque des revendications 1 à 15, l'administration du composé à un être humain réduisant la concentration plasmatique de Lp(a) à jeun chez l'être humain d'au moins 50 %, d'au moins 75 %, d'au moins 80 % ou d'au moins 85 %, lorsque la concentration plasmatique de Lp(a) à jeun chez l'être humain est mesurée au début et à la fin de la période de traitement.
